# EUROPEAN PATENT SPECIFICATION

(11) **EP 3 615 661 B1**
(45) Date of publication and mention of the grant of the patent: **11.02.2026**
(21) Application number: 18720229.6
(22) Date of filing: 25.04.2018
(51) Int. Cl.: C12N 5/071, C12N 5/079

(54) **BI- OR MULTI-DIFFERENTIATED ORGANOID**
BI- ODER MULTI-DIFFERENZIERTES ORGANOID
ORGANOÏDE BI OU MULTI-DIFFÉRENCIÉ

(30) Priority: 25.04.2017 EP 17168051
(43) Date of publication of application: 04.03.2020
(73) Proprietor: IMBA-Institut für Molekulare Biotechnologie GmbH, 1030 Wien (AT)
(72) Inventor: KNOBLICH, Jürgen, 1030 Vienna (AT); BAGLEY, Joshua A., 1030 Vienna (AT)
(74) Representative: SONN Patentanwälte GmbH & Co KG
(86) International application number: PCT/EP2018/060559
(87) International publication number: WO 2018/197544

(56) References cited:
- WO-A1-2011/055855
- WO-A1-2014/033322
- WO-A1-2014/090993
- WO-A1-2018/183446
- US-A1- 2016 289 635
- US-A1- 2016 312 181
- MADELINE A. LANCASTER ET AL: "Cerebral organoids model human brain development and microcephaly", NATURE, vol. 501, no. 7467, 28 August 2013 (2013-08-28), pages 373 - 379, XP055166627, ISSN: 0028-0836, DOI: 10.1038/nature12517
- M. A. LANCASTER ET AL: "Organogenesis in a dish: Modeling development and disease using organoid technologies", SCIENCE, vol. 345, no. 6194, 17 July 2014 (2014-07-17), pages 283,1 - 9, XP055130123, ISSN: 0036-8075, DOI: 10.1126/science.1247125
- JOSHUA A BAGLEY ET AL: "Fused cerebral organoids model interactions between brain regions", NATURE METHODS, 1 January 2017 (2017-01-01), XP055386037, ISSN: 1548-7091, DOI: 10.1038/nmeth.4304

## Description

The present invention relates to the field of artificial organoid model tissues.

### Background of the invention

Three-dimensional (3D) organoid culture technology allows the development of complex, organ-like tissues reminiscent of *in vivo* development (WO2014/090993; Lancaster et al., Nature 501, 373-379 (2013); Lancaster & Knoblich, Science 345, 1247125 (2014)). Importantly, cerebral organoids recapitulate many aspects of embryonic cortical development including the generation of diverse cell types corresponding to different brain regional identities (Lancaster & Knoblich, Nat Protoc 9, 2329-2340 (2014)). For instance, cerebral organoids can produce dorsal and ventral forebrain progenitors that generate excitatory neurons and inhibitory interneurons, respectively. Moreover, cerebral organoids can be generated from human patient-derived induced pluripotent stem cells (hiPSCs), and used for functional genomic studies of neurological disorders such as microcephaly and Autism.

Maroof et al., Cell Stem Cell 12, 559-572 (2013) and Nicholas et al., Cell Stem Cell 12, 573-586 (2013) describe methods of modelling human neuronal development in mice *in vivo.* The method comprises culturing stem cells on a cortical feeder layer *in vitro* and then transplanting cells into the neonatal neocor-tex of immunocompromised mice for further development. Liu et al., Nat Protoc 8, 1670-1679 (2013) describe culturing cells into small neurospheres (20µm-50µm) and differentiating the cells of the neurospheres into GABA⁺ neurons in culture.

US 2016/289635 A1 describes to a method of producing an artificial telencephalon tissue.

WO 2014/033322 A1 relates to a method of producing pancreatic endoderm by differentiating precursor cells.

WO 2011/055855 A1 relates to a method of inducing differentiation in cultured stem cells.

US 2016/312181 A1 describes differentiation of neuronal cells from stem cells.

Still, such prior model systems are not able to simulate any physiological development. E.g. the cerebral cortex contains two main populations of neurons, excitatory glutamatergic pyramidal neurons, and inhibitory γ-Aminobutyric acid (GABA) producing interneurons. Excitatory cortical neurons are predominantly generated by dorsal forebrain progenitors, while inhibitory GABAergic cortical interneurons are generated by ventral forebrain progenitors. To integrate into cortical circuits, interneurons perform a long-distance migration from their ventral origin into their target dorsal cortical regions. This long-range tangential migration is controlled by many signaling pathways, and mutations in some neurological disease-associated genes may disrupt interneuron migration. However, the relationship between patient-specific mutations and human brain development remains enigmatic without suitable experimental human model systems.

Thus, there is a demand to model additional characteristics of natural, in particular human, brains in large *in vitro* cultures that closely resemble brains and model brain development to a stage as late/large as possible.

### Summary of the invention

The present invention provides an in vitro method of producing a bi- or multi-differentiated neuronal tissue with at least two neuronal tissue types comprising the steps of providing a first neuronal tissue that is developed to a stage of differentiation of interest; providing a second neuronal tissue that is developed to a stage of differentiation of interest differing from the stage of differentiation of interest of the first neuronal tissue; placing said first and second neuronal tissue in vicinity sufficient for fusion by growth; allowing the first and second neuronal tissue to grow and fuse to each other; thereby producing a bi- or multi-differentiated neuronal tissue comprising the first and second tissue with different stages of differentiation.

Also provided is an in vitro method of producing a bi- or multi-differentiated neuronal tissue with at least two neuronal tissue types comprising the steps of developing a first neuronal tissue to a stage of differentiation of interest; developing a second neuronal tissue to a stage of differentiation of interest differing from the stage of differentiation of interest of the first neuronal tissue; placing said first and second neuronal tissue in a vicinity sufficient for fusion by growth; allowing the first and second neuronal tissue to grow and fuse to each other; thereby producing a bi- or multi-differentiated neuronal tissue comprising the first and second tissue with different stages of differentiation.

The invention further provides a bi- or multi-differentiated neuronal tissue with at least two neuronal tissue types of different stages of differentiation , wherein at least one of the neuronal tissue types comprises ventral neuronal tissue and the at least another tissue type is substantially non-ventral but containing migrated cells from the ventral neuronal tissue constituting not more than 5% of the cells of the substantially non-ventral tissue and the bi- or multi-differentiated neuronal tissue having a size of 100 µm to 10 mm in its longest dimension.

Further provided is a method of testing a candidate compound for influencing development of bi- or multi-differentiated neuronal tissue, comprising contacting cells or a neuronal tissue in a method of the invention with the candidate compound or contacting a neuronal tissue of the invention with the candidate compound and maintaining said contacted tissue in culture, and observing any developmental changes in the tissue as compared to said tissue without contacting by said candidate compound.

Also provided is a use of a kit in a method of the invention, the kit comprising a ventral differentiation inducer and a dorsal differentiation inducer, preferably a WNT inhibitor and/or a SHH enhancer as ventral differentiation inducer and preferably a SHH inhibitor as dorsal differentiation inducer. The kit is useful for and is used in a method of the invention.

All embodiments of the invention are described together in the following detailed description and all preferred embodiments relate to all embodiments, aspects, methods, tissues and kits alike. E.g. kits or their components can be used in or be suitable for inventive methods. Any component used in the described methods can be in the kit. Preferred and detailed descriptions of the inventive methods read alike on suitability and resulting tissues of the inventions. All embodiments can be combined with each other, except where otherwise stated.

### Detailed description of the invention

The present invention provides an in vitro method of producing a bi- or multi-differentiated neuronal tissue with at least two neuronal tissue types comprising the steps of providing or developing a first neuronal tissue at/to a stage of differentiation of interest; providing or developing a second neuronal tissue at/to a stage of differentiation of interest differing from the stage of differentiation of interest of the first neuronal tissue; placing said first and second neuronal tissue in a vicinity sufficient for fusion by growth; allowing the first and second neuronal tissue to grow and fuse to each other; thereby producing a bi- or multi-differentiated neuronal tissue comprising the first and second tissue with different stages of differentiation. The method is performed *in vitro* or *ex vivo,* such as in a container like a vial or dish or other cell culture equipment. Typically, the first and/or the second neuronal tissue, especially both (and optionally further tissue), are derived in-vitro, i.e. from an in-vitro culture. Preferably, the first and/or the second tissue, especially both (and optionally further tissue), are derived in vitro from pluripotent stem cells or, in other words, from an in-vitro culture of pluripotent stem cells. It is particularly preferred when the first and/or the second neuronal tissue, especially both (and optionally further tissue), are derived from induced pluripotent stem cells (which in turn can e.g. be derived from cells isolated from a patient) or, in other words, derived from an in-vitro culture of induced pluripotent stem cells.

Artificial tissue culturing methods, including 3D-culturing, are known from WO2014/090993, Lancaster et al., Nature 501, 373-379 (2013), Lancaster & Knoblich, Science 345, 1247125-1247125 (2014) and Lancaster & Knoblich, Nat Protoc 9, 2329-2340 (2014). Such prior methods can be used according to the invention for developing the first and second and any further tissue that form part of the inventive bi- or multi- differentiated tissue. PCT/EP2017/050469 (WO2017/121754) describes an improved method of creating organoids on support in a three dimensional matrix. Such a method may also be used according to the invention. Also US 2011/0143433 describes culturing stem cells that may develop into tissues, which may be used according to the invention. Prior organoids succeeded in creating artificial *in vitro* neuronal tissues with natural brain like characteristics and layering.

In summary, many tissues have been developed. Such tissues, may be used as starting points for the invention as developed first neuronal tissue at a given stage of differentiation of interest and as a second (and optionally any further) neuronal tissue at a given stage of differentiation of interest. Of special interest are neural or neuronal tissues. Neural tissue undergoes complex differentiation pattern and correct modelling of human brains pose a difficulty for developmental biology. Although advances have been made (all papers by Lancaster et al. and Lancaster & Knoblich, supra), modeling full development of a tissue that can evolve into an animal or human brain is still beyond reach (Brüstle, Nature 501 (2013): 319-320; Chambers et al., Cell Stem Cell (2013) 13: 377-378). The inventive method and tissues relate to neuronal or neural tissues since for these tissues particular advantageous effects and results have been shown.

The cells of the first, second and optionally further neuronal tissue may be developed to a particular organ type or may have initiated differentiation towards a particular differentiation fate. The cells of the tissues may have become multipotent (from previously pluripotent some cells may even be unipotent or somatic tissue cells. The tissue fate may be towards any tissue. Preferably, the target tissue is selected from neuronal tissue. For example the tissues may comprise any stem cell for such a tissue that has undergone tissue specific differentiation. Preferably the tissues comprise cells selected from neuronal or neurogenic cells. In some cases, also combinations are possible, e.g. organ cells (e.g. neuronal) with cells that would develop into supporting tissues (e.g. endothelial, adipogenic, ligamentogenic cells). In the methods, differentiation may be initiated by commonly known tissue specific growth or differentiation factors, also called, differentiation-inducing agents. Such are e.g. known in the art and are e.g. disclosed in WO 2009/023246 A2, WO 2004/084950 A2 and WO 2003/042405 A2. Further, the differentiating or growth factor can be an insulin-like growth factor, a nerve growth factor, a neurotrophin, a growth hormone, an interleukin. These factors/agents are commercially available and need no further description. Of course, such factors/gents may for any one of the above tissue types may be included in the inventive kit. Preferably, neuronal or neurogenic are used in the method or provided in the kit and preferably neuronal or neurogenic cells are present in the inventive neuronal tissue.

The tissue may comprise progenitor cells, such as a stem cell, to any tissue, especially those described above. The progenitor cell is preferably selected from the group consisting of pluripotent stem cell, multipotent stem cell, mesenchymal stem cell, neuronal stem cell, embryonic stem cell, neural stem cell, especially a neural crest stem cell. The pluripotent cell used in the method or the progenitor cell can be derived from a de-differentiated chondrogenic cell, myogenic cell, osteogenic cell, tendogenic cell, ligamentogenic cell, adipogenic cell, or dermatogenic cell.

Differentiation can be achieved by contacting cells with a tissue specific growth or differentiation factor. The cells may then develop into the desired tissue. Such a tissue specific growth or differentiation factor may be a neuronal or neurogenic, myogenic, tenogenic, chondrogenic, or osteogenic differentiation factor, especially preferred a neuronal differentiation factor. This will determine the development into the respective type of cellular tissue in later development. The cells will thereby transit from pluripotent to multipotent cells. Other tissue types shall then be not or only by a return to a pluripotent status be possible again. Usually not all cells are differentiated to the selected tissue type. It usually sufficient when about 30% or more or at least 40% or at least 50%, or at least 60% or at least 70% or at least 80% of the cells initiate differentiation towards the selected tissue type and transform to reduce their differentiation potential by multipotent cell with the respective tissue destiny (%-values as fractions of the cell amount). Of course this differentiation destiny only applies for the cells that are not returned to a un- or less differentiated state by use of artificial growth and dedifferentiation stimuli. Clearly, even somatic cells can be returned to a pluripotent cells and this is not meant when defining a differentiated state herein. Preferably, no factors are introduced to the cells that would return the cells to pluripotent cells.

The inventive first, second and optionally further neuronal tissues have different stages of development or differentiation. Such different differentiation means that the tissues have at least initiated differentiation into different neuronal tissue types, including subtypes or other different organ regions or organ organization areas, such as ventral and dorsal development and/or development of different brain areas and/or development of left and right brain hemispheres .

The first, second and optionally further neuronal tissues are then placed in vicinity and allowed to grow together or fuse. This surprisingly led to remarkable new observations in development of the now combined or fused tissue (also referred to as bi- or multi-differentiated tissue). For example, development of the forebrain involves the migration of GABA-ergic interneurons over long distances from ventral into dorsal regions. Although defects in interneuron migration are implicated in neuropsychiatric diseases such as Epilepsy, Autism, and Schizophrenia, model systems to study this process in humans are still lacking. An organoid co-culture "fusion" paradigm to successfully combine independently patterned organoids into a single tissue was developed. The inventive bi- or multi-differentiated neuronal tissue not only achieves the benefits of prior organoid models but on top of that is suitable to provide a model for cell migration between tissue types, such as in human interneuron migration. In case of cerebral organoids, by fusing organoids specified toward dorsal and ventral forebrain, a continuous dorsal-ventral axis has been shown. Using fluorescent reporters, directional GABAergic interneuron migration from ventral into dorsal forebrain can be monitored. The molecular taxonomy and migratory dynamics of these cells resembles that of cortical interneurons. Time lapse imaging of human interneuron migration is possible. Also inhibition of such migration, e.g. by the CXCR4 antagonist AMD3100 is observable. The results demonstrate that cerebral organoid fusion cultures can model complex interactions between different brain regions. Combined with reprogramming technology, fusions offer a possibility to analyze complex neu-rodevelopmental defects using cells from neuropsychiatric disease patients, and to test potential therapeutic compounds.

Cell co-cultures are known in the art. E.g. stem cells are cultured in mixtures with other cells to provide additional support or developmental factors to the stem cells (Paschos et al., Tissue Eng Regen Med (2014) DOI: 10.1002/term.1870). The present invention relates to a different kind of co-culture. The invention provides a co-culture on the tissue level, wherein two or more differently developed tissues are cultured together to form a fusion tissue, the bi- or multi-differentiated tissue. Individual cells of the different original tissues are not mixed by the practitioner, as the original different tissues remain separate regions of tissue types within the bi- or multi-differentiated tissue. However, cell migration is of course allowed. In fact natural migration of cells from one tissue to another is desired and shows natural development of fused tissues. Some cells form connections between the different tissue types.

Growth of the tissues to form the bi- or multi-differentiated neuronal tissue may be performed as known in the art for tissue culturing. Preferred is culturing in a three dimensional matrix. A three dimensional matrix is distinct from 2D cultures, such as 2D cultures in a dish on a flat surface. A "three dimensional culture" means that the culture can expand in all three dimensions without being blocked by an one-sided wall (such as a bottom plate of a dish). Such a culture is preferably in suspension. The three dimensional matrix may be a gel, especially a rigid stable gel, which results in further expansion of growing cell culture/tissue and differentiation. A suitable three dimensional matrix may comprise collagen. More preferably the three dimensional matrix comprises extracellular matrix (ECM) or any component thereof selected from collagen, laminin, entactin, and heparin-sulfated proteoglycan or any combination thereof. Extra-cellular matrix may be from the Engelbreth-Holm-Swarm tumor or any component thereof such as laminin, collagen, preferably type 4 collagen, entactin, and optionally further heparan-sulfated proteoglycan or any combination thereof. Such a matrix is Mat-rigel. Matrigel is known in the art (US 4,829,000) and has been used to model 3D heart tissue previously (WO 01/55297 A2) or neuronal tissue (WO 2014/090993). Preferably the matrix comprises laminin, collagen and entactin, preferably in concentrations 30%-85% laminin, 3%-50% collagen and sufficient entactin so that the matrix forms a gel, usually 0.5%-10% entactin. Laminin may require the presence of entactin to form a gel if collagen amounts are insufficient for gel forming. Even more preferred, the matrix comprises a concentration of at least 3.7 mg/ml containing in parts by weight about 50%-85% laminin, 5%-40% collagen IV, optionally 1%-10% nidogen, optionally 1%-10% heparan sulfate proteoglycan and 1%-10% entactin. Matrigel's solid components usually comprise approximately 60% laminin, 30% collagen IV, and 8% entactin. All %-values given for the matrix components are in wt.-%. Entactin is a bridging molecule that interacts with laminin and collagen. Such matrix components can be added in step r). These components are also preferred parts of the inventive kit. The three dimensional matrix may further comprise growth factors, such as any one of EGF (epidermal growth factor), FGF (fibroblast growth factor), NGF, PDGF, IGF (insulin-like growth factor), especially IGF-1, TGF-β, tissue plasminogen activator. The three dimensional matrix may also be free of any of these growth factors.

In general, the three dimensional matrix is a three dimensional structure of a biocompatible matrix. It preferably comprises collagen, gelatin, chitosan, hyaluronan, methylcellulose, laminin and/or alginate. The matrix may be a gel, in particular a hydrogel. Organo-chemical hydrogels may comprise polyvinyl alcohol, sodium polyacrylate, acrylate polymers and copolymers with an abundance of hydrophilic groups. Hydrogels comprise a network of polymer chains that are hydrophilic, sometimes found as a colloidal gel in which water is the dispersion medium. Hydrogels are highly absorbent (they can contain over 99 wt.-% water) natural or synthetic polymers. Hydrogels also possess a degree of flexibility very similar to natural tissue, due to their significant water content. It is possible that the three dimension-al matrix, or its components, especially ECM or collagen, still remains in the produced tissue culture. Preferably the three dimensional matrix is a collagenous matrix, preferably it contains type I and/or type IV collagen.

The first, second and optionally further neuronal tissues that are used as a starting material for the fusion, may also be grown or differentiated in a three dimensional matrix as described above - of course suitable growth factors may be used for differentiation into the desired tissue type as mentioned above. Accordingly, the present invention also provides the steps of growing cells, preferably stem cells, in a three dimensional matrix and differentiating cells into the first and/or second and/or further neuronal tissues that are to be used further in the inventive method. The present invention also provides using tissues that have been grown in a three dimensional matrix.

Preferably, developing the first neuronal tissue to a stage of differentiation of interest is separate from developing the second neuronal tissue to a stage of differentiation of interest differing. Separating the differentiation of the first and second tissue (and also of further tissues if indented to be used) allows better control of the differentiation. Of course also co-differentiation is possible, at least to a certain stage, e.g. towards a general organ destination, like neuronal tissue in general or of any other organ or tissue class as mentioned above, with separate differentiation towards particular tissue subtypes, like dorsal or ventral tissues. Separate differentiation can be e.g. in separate containers, e.g. different vials, flasks or dishes.

Disclosed is an artificial neuronal tissue culture comprising a first and/or second neuronal tissue. Such a culture may be *in vitro* grown from a cell or from an aggregate of cells. The culture is in particular not a culture of an *in vivo* developed brain or a tissue sample thereof. The first and second neuronal tissues, used as a starting block to generate the invention, are preferably separable, i.e. not already grown together. They can be separated without disconnecting cellular or ECM (extracellular matrix) bonds, such as by cutting or tearing.

The first and second neuronal tissues are then placed in vicinity that allows fusion by growth. The tissues may be placed directly adjacent or with little room in between (e.g. filled by a matrix material, e.g. of the three dimensional matrix mentioned above) that allows joining of the tissues by growth. Such a distance may be 0.001 µm to 50 µm, preferably 0.01 µm to 20 µm or 0.1 µm to 10 µm or up to 1 µm. The steps of placing said first and second neuronal tissue in a vicinity sufficient for fusion by growth and allowing the first and second tissue to grow and fuse to each other are preferably performed in suspension culture, e.g. after placement in an adequate culturing medium or matrix, such as a 3D matrix like a hydrogel.

During joining and also afterwards, the neuronal tissues are preferably cultured in a three dimensional matrix. Accordingly, the first and second (and optionally further) tissues are placed inside a three dimensional matrix material as described above, preferably a hydrogel. The tissues will then continue to grow and fuse to form the bi- or multi-differentiated tissue. The bi- or multi-differentiated tissue many be continued to grow or be maintained in a three dimensional matrix, such as a hydrogel for further development. Such further development may include allowing migration of cells from one tissue type to another tissue type. The tissue types are those tissues that have been given rise by the original separate first and second tissues (and optionally further tissues if present). Thus in a preferment the invention comprises migrating cells from the first tissue to the second tissue in said bi- or multi-differentiated tissue.

Preferably the neuronal tissue in cultured further even after joining (fusion). After joining, interesting processes, like cell migration between tissue types, may be observed. Further development may take place. Preferably the bi- or multi-differentiated neuronal tissue is cultured for at least 5 days after joining, preferably at least 10 days after joining.

The tissues are neuronal tissues, particularly, the inventive bi- or multi-differentiated neuronal tissue is a cerebral organoid. Cerebral organoids are e.g. disclosed in WO2014/090993, PCT/EP2017/050469, Lancaster et al., Lancaster & Knoblich, all supra. An organoid is a miniaturized and simplified version of an organ produced in vitro in three dimensions that shows realistic micro-anatomy. They are derived from one or a few cells from a tissue, embryonic stem cells or induced pluripotent stem cells, which can self-organize in three-dimensional culture owing to their self-renewal and differentiation capacities. The first, second and optionally further tissue may already be organoids or are capable of developing into organoids, given the corresponding growth or differentiation factor stimulus. Preferably at least the bi- or multi-differentiated neuronal tissue is grown into an organoid, which then is a bi- or multi-differentiated organoid.

In preferments, the at least the first neuronal tissue comprises outer radial glia, a cortical subventricular zone and a cortical inner fiber layer (and/or comprising neural progenitor cells and neurons). Alternatively or in addition, especially in addition, it is preferred that at least the second neuronal tissue comprises outer radial glia, a cortical subventricular zone and a cortical inner fiber layer (and/or comprising neural progenitor cells and neurons). These regions, zones and layers are hallmarks of advanced developed cerebral organoids and are preferably present in order to study advanced neural development. Preferably, the bi- or multi-differentiated neuronal tissue comprises outer radial glia, a cortical subventricular zone and a cortical inner fiber layer (and/or comprising neural progenitor cells and neurons).

Preferably, the stages of differentiation of the first neuronal tissue comprises a ventral forebrain progenitor tissue or a rostro-ventral forebrain tissue. This differentiation into a ventral or ventral-like tissue type may be initiated by WNT-inhibition and/or SHH signalling enhancement. A suitable WNT-inhibitor that can be used to treat the cells of the first tissue is IWP2 ("Inhibitor of WNT Production-2", e.g. CAS-no: 686770-61-6). A suitable SHH signalling enhancer that can be used to treat the cells of the first tissue is SAG ("Smoothened Agonist", e.g. CAS-no: 364590-63-6). Concentrations as described in the examples may be used. WNT-inhibition and/or SHH signalling enhancement is/are preferably concurrent with neural induction, i.e. differentiation progenitor cells toward neural tissue development. Likewise preferably, the stages of differentiation of the second tissue comprises dorsal forebrain tissue or neuroectoderm without differentiation to ventral or dorsal forebrain. Differentiation into a dorsal or dorsal-like tissue type may be initiated by SHH activity inhibition. A suitable SHH activity inhibitor that can be used to treat the cells of the second tissue is cyclopamine A (CycA). Concentrations as described in the examples may be used. SHH inhibition is preferably concurrent with neural induction, i.e. differentiation progenitor cells toward neural tissue development. Preferably, the bi- or multi-differentiated tissue comprises a ventral forebrain progenitor tissue or a rostro-ventral forebrain tissue at one tissue region - corresponding to the first tissue -, and dorsal forebrain tissue or neuroectoderm without differentiation to ventral or dorsal forebrain in another region - corresponding to the second tissue. Briefly, ventral and dorsal tissues allow observations of advanced neural developments, such as migrating neural cells, in particular of GABA-ergic neuronal cells, such as those forming interneurons. Such cells may migrate out of a ventral region, which is therefore preferably present in the inventive first tissue and/or in the bi- or multi-differentiated neuronal tissue.

Preferably, the bi- or multi-differentiated neuronal tissue is differentiated to develop a neural plate or further to a neural tube. A neural plate or tube may be present in the inventive bi- or multi-differentiated neuronal tissue.

In preferred embodiments of the invention, the first neuronal tissue is grown to a size of at least 100 µm, preferably at least 150 µm, especially preferred at least 200 µm. Likewise preferably, the second neuronal tissue is grown to a size of at least 100 µm, preferably at least 150 µm, especially preferred at least 200 µm. Preferably the sizes of the first and second neuronal tissue are grown to about the same size before allowed to fuse to form the bi- or multi-differentiated neuronal tissue. About the same size means that the volume differs by at most 40%, preferably b at most 30%, at most 20% or at most 10%. "Size" refers to the longest dimension in 3d space. Preferably the neuronal tissues are globular in shape, in particular with the shortest dimension being not less than 20% of the longest dimension, in particular not less than 30% or not less than 40% of the longest dimension. Preferably the volume of the first and/or second tissue is at least 1x10⁶ µm³, in particular preferred at least 2x10⁶ µm³, at least 4x10⁶ µm³, at least 6x10⁶ µm³. The bi- or multi-differentiated neuronal tissue may have the same sizes, shapes and volumes or even larger sizes and volumes, such as a volume of at least 8x10⁶ µm³, at least 10x10⁶ µm³, at least 15x10⁶ µm³ and/or sizes of at least 250 µm, especially preferred at least 350 µm.

The first and second neuronal tissues are usually small aggregates of cells and may have a size of at most 2 mm, preferably of at most 1250 µm or at most 800 µm, e.g. with volumes of at most 8 mm³, at most 2 mm³, or at most at most 1 mm³. In some embodiments, the bi- or multi-differentiated neuronal tissue may be larger with a size of at most 15 mm, preferably of at most 10 mm or at most 5 mm, e.g. with volumes of at most 60 mm³, at most 30 mm³, or at most at most 10 mm³.

The first and second neuronal tissue may express certain differentiation expression markers, or lack expression of such expression markers as signals of a specific differentiation. Expression markers are signs of different differentiation of these two tissues.

Preferably the inventive first neuronal tissue and/or bi- or multi-differentiated neuronal tissue comprises cells, which express DLX2. DLX2 is expressed in cells of ventral forebrain identity. Preferably this tissue type is comprised in the inventive tissue.

Preferably the inventive first neuronal tissue and/or bi- or multi-differentiated neuronal tissue comprises cells, which express GSX2. GSX2 is expressed in cells of dorsal-lateral ganglionic eminence and caudal ganglionic eminence identity. Preferably this tissue type is comprised in the inventive tissue.

Preferably the inventive first neuronal tissue and/or bi- or multi-differentiated neuronal tissue comprises cells, which express NKX2-1. NKX2-1 is expressed in cells of ventral-medial ganglionic eminence identity. Preferably this tissue type is comprised in the inventive tissue.

Preferably the inventive first neuronal tissue and/or bi- or multi-differentiated neuronal tissue comprises cells, which express LHX6. LHX6 is expressed in cells of a subregion of ventral-medial ganglionic eminence identity. Preferably this tissue type is comprised in the inventive tissue.

Preferably the inventive first neuronal tissue, second tissue and/or bi- or multi-differentiated neuronal tissue comprises cells, which express FoxG1. FoxG1 is expressed in cells of dorsal cortex identity. Preferably this tissue type is comprised in the inventive tissue.

Preferably the inventive second neuronal tissue and/or bi- or multi-differentiated neuronal tissue comprises cells, which express TBR1. TBR1 is expressed in cells of dorsal forebrain identity. Preferably this tissue type is comprised in the inventive tissue.

Preferably the inventive second neuronal tissue and/or bi- or multi-differentiated neuronal tissue comprises cells, which express TBR2. TBR2 is expressed in cells of dorsal cortical identity. Preferably this tissue type is comprised in the inventive tissue.

The inventive bi- or multi-differentiated neuronal tissue may contain a region of fusion between the tissue types reminiscent of the first and second tissue that gave rise to them. Such a region of fusion may allow the regions of the tissue types to be juxtaposed, resulting in a tissue that is a continuous tissue where one side is of the first tissue type, while the other side is of the second tissue type.

Preferably, the first and/or second neuronal tissue expresses a detectable marker that is not expressed by the other from the group of the first and second neuronal tissue. The same applies to further tissues, if present, in preferred embodiments. The detectable marker should not be confused with the expression marker of the preceding paragraph. The detectable markers here refer to a label that is introduced, such as for recombinant expression, into the cells of the first and/or second neuronal tissue as a label of said tissue. The emphasis is easy detection and measurement, such as in case of fluorescence markers. The detectable makers preferably provide an optical signal, such as a colour or fluorescence. The detectable marker may be used to track cells of the first or second neuronal tissue origin (tissue types) throughout the bi- or multi-differentiated neuronal tissue. As said, cells may migrate and travel between tissue types.

Disclosed is a bi- or multi-differentiated neuronal tissue obtainable by a method of the invention. According to the inventive method, when performed *in vitro,* then the tissue is also provided *in vitro.* Alternatively, it may be implanted into a non-human animal, such as a rodent.

The invention provides a bi- or multi-differentiated neuronal tissue with at least two neuronal tissue types of different stages of differentiation, wherein at least one of the neuronal tissue types comprises ventral neuronal tissue and the at least another neuronal tissue type is substantially non-ventral but containing migrated cells from the ventral neuronal tissue constituting not more than 5% of the cells of the substantially non-ventral tissue. Also this tissue may be provided *in vitro* or implanted into a non-human animal, such as a rodent. *In vitro* means the same as mentioned above, preferably the tissue is provided in a container such as a vial. It may be provided in suspension or suspendable, i.e. not fixed to a wall of the container.

Since the inventive neuronal tissue is the product of fusion of different neuronal tissues according to the use of tissues that are developed to different stages of differentiation, the inventive bi- or multi-differentiated neuronal tissue will reflect and comprise these different tissue fusions. Preferred are any stages of development as mentioned above, e.g. ventral or dorsal developed tissues. Other embodiments provide the fusion of healthy and diseased stages of development, such as diseases selected from neuropsychiatric diseases such as Epilepsy, Autism, and Schizophrenia as mentioned above. Also, the fusion of two different diseased states is possible. Such fusions show characteristic cell migration or impairment thereof according to the disease. Migration can be monitored in the other (usually healthy) tissue. Healthy means non-diseased state. Preferably the three dimensional matrix, such as a hydrogel, is still present in the bi- or multi-differentiated tissue.

Preferably the non-human animal for implantation is immunocompromised in order to avoid tissue rejection.

Such a neuronal tissue constitutes a neuronal tissue wherein a ventral differentiated first tissue is fused with another not-ventral differentiated tissue, such as a dorsal differentiated second tissue or a neuronal differentiated tissue (without further ventral differentiation) is fused as discussed in more detail above. Of course, all preferred embodiments described above also relate to this tissue, especially the regions, sizes and detection marker or expression marker, etc. embodiments. E.g. at least one of the tissue types expresses a detectable marker, preferably a fluorescence marker, not expressed by another tissue type of the bi- or multi-differentiated tissue. This allows easy detection of said tissue and its cells, which may migrate to other tissue types. Preferably the tissue type with the marker is the ventral differentiated first tissue.

The provided neuronal tissue may have migrating or migrated cells. Such cells may have moved to another tissue type that is substantially non-ventral (e.g. dorsal). The migrated cells from the ventral neuronal tissue in the non-ventral tissue may constitute not more than 5% of the cells of the substantially non-ventral tissue (% of cell amounts of the non-ventral/second tissue). Preferably 5% or less, 4% or less, 3% or less, 2% or less or 1% or less or ventral cells, or generally cells of the first tissue are found in the non-ventral tissue or generally second or optionally further tissue (% in cell amounts of the tissue wherein the ventral/first tissue cells are found).

The bi- or multi-differentiated neuronal tissue preferably has a size of 100 µm to 10 mm in its longest dimension, or any size, shape or volume mentioned already above. Preferred is as size of 250 µm to 10 mm or 500 µm to 5 mm.

The inventive bi- or multi-differentiated neuronal tissue may be provided in form of a globular body, e.g. as described above in any of the given shapes or aspect rations (dimension ratios). The bi- or multi-differentiated neuronal tissue may also be provided as a tissue slice. A bi- or multi-differentiated neuronal tissue may be grown as detailed above and then a tissue slice may be obtained. Such a slice may have a thickness of e.g. 3 µm to 100 µm or 5 µm to 50 µm or 10 µm to 30 µm, preferably about 20 µm. Other dimensions, e.g. the longest as mentioned above, may still be exhibited by the slice. Slicing is preferably performed in frozen state. Slices may be used to detect detection and/or expression markers in the tissue slice. Slices may be used for microscopic imaging.

The neuronal tissue is considered an artificial tissue. "Artificial" means that it is grown *in vitro* and has certain characteristics of artificial cultures, like size, consistency, shape and cell organization. The shape may be irregular and different from natural occurring tissues and cell organization may differ due to size restrains. In particular, "artificial" excludes naturally occurring tissues and organs and their parts, e.g. natural tissue slices. The three dimensional matrix may be still in the culture and/or the artificial tissue may have the shape determined by growth in such a matrix. E.g. the culture may be obtainable by growth in a three dimensional matrix, especially those as described above. Preferably, the inventive tissue is an organoid, especially a cerebral organoid).

The neuronal tissue may be of any neuronal tissue type mentioned above and may comprise any of the cells, e.g. stem cells mentioned above.

The invention provides an organoid fusion assay that allows analysis of human cortical interneuron migration. This technology enhances the repertoire of phenotypic assays available for cerebral organoids, and in turn the complexity of phenotypes that can be used to study the developmental cell biology of human neurological diseases.

E.g. a method of testing or screening a candidate compound for influencing development of bi- or multi-differentiated neuronal tissue is provided, said method comprises contacting cells or a tissue in a method of any one of the invention with the candidate compound or contacting a tissue of the invention with the candidate compound and maintaining said contacted tissue in culture, and observing any developmental changes in the tissue as compared to said tissue without contacting by said candidate compound.

The contacting step is a treating step of cells to be developed into the inventive neuronal tissue or of a neuronal tissue or its precursor tissues (like the first, second and/or any further tissues). The candidate compound may be a small organic molecule, such as molecules with a mass of 100 Da to 5000 Da. Other candidate compounds may be biomolecules such as proteins, nucleic acids or carbohydrates. Further candidate compounds may be bulk chemicals such as solvents, like ethanol - of course used in concentrations generally viable for cells - or polymers. The treatment should be in a concentration wherein a specific effect of the compound can be expected. Various concentrations may be tested in parallel. Usually concentration of candidate compounds is 1 ng/ml to 100 mg/ml, e.g. of 100 ng/ml to 1 mg/ml.

In particular preferred, the inventive methods or tissues can be used to test for side effects in drug candidates as candidate compounds. Preferably, causes, the nature and possible cures for congenital disorders are investigated by monitoring for any changes. For example, teratogenic effects can be tested by monitoring the development and/or growth of the inventive tissue when contacted by a potentially causative substance/environmental change/genetic modification.

By this method, developmental effects or disorders influenced by candidate compounds or other environmental influences can be investigated. If an effect is only expected during initial differentiation, e.g. when differentiating and growing a cell to the first/and or second or optionally further neuronal tissue, it is sufficient to contact the cell. If an effect is expected during later differentiation and/or cell arrangement or growth after differentiation (e.g. an organ specific teratogen), contacting the first and or second or optionally further neuronal tissues may be sufficient. It is also possible to contact the tissues after or during fusion. Of course, this can be combined with contacting during cell differentiation. Such a teratogenic compound is for example ethanol, which leads to fetal alcohol disorders such as fetal alcohol syndrome if an embryo or fetus is exposed to it during development. The effects of ethanol on developing tissues, e.g. brain tissues during development can be investigated with the inventive method.

According to one preferment, the invention provides the method of investigating a developmental tissue effect comprising i) decreasing or increasing the expression in a gene of interest in a cell or ii) administering a candidate drug to the cells, at any stage during the inventive method, wherein said cell is cultured towards the of bi- or multi-differentiated neuronal tissue according to the invention, preferably during differentiation to the neuronal tissue types and/or fusion.

Also provided is a method of screening a candidate therapeutic agent suitable for treating a developmental tissue defect of interest, comprising performing the inventive differentiation and/or fusion method and administering the candidate agent to said cells at any stage during the method (as above), preferably at all stages.

Also provided is a method of testing a candidate drug for developmental effects, especially for congenital disorder effects, comprising administering a candidate drug to a tissue according to the invention or during the methods (e.g. during differentiation, development of the first/second neuronal tissue, during fusion or after fusion) and determining an activity of interest of the cells of said tissue and comparing said activity to an activity of cells of a tissue without administering said candidate drug, wherein a differential activity (to a control) indicates a developmental effect.

Further provided is a use of a kit of compounds and substances in an inventive method. The kit comprises means to perform any of the inventive methods. Of course not all substances need to be included since some are standard chemicals or usually available. Nevertheless, preferably the core substances are provided. In other kits, rarer substances are provided. Of course the inventive kits or their substances may be combined. Components in the kit are usually provided in separate containers, such as vials or flasks. Containers may be packaged together. Preferably the kit comprises a manual or instructions for performing the inventive methods or their steps.

Provided is a use of a kit in a method according to the invention. The kit comprises a ventral differentiation inducer and a dorsal differentiation inducer, preferably a WNT inhibitor and/or a SHH enhancer as ventral differentiation inducer and preferably a SHH inhibitor as dorsal differentiation inducer. Also possible are neural growth or differentiation factors without ventral or dorsal differentiation activity. Such a kit may be used for neural differentiation, e.g. to generate cerebral organoids.

Preferably, the kit comprises a three dimensional matrix as described above, or their components to generate such a three dimensional matrix. Matrix components may be provided in solid state, such as lyophilized state to be reconstituted to the matrix, e.g. by hydration. Any matrix or their components as described above may be included in the kit. Preferably the matrix is a hydrogel, especially a collagenous hydrogel as described above. The kit may comprise such reconstitutable (preferably collagenous) components. Further preferred matrix components are carbohydrates, in particular in polymeric form (polysaccharides). A preferred polysaccharide is agarose.

Any kit may further comprise cell growth nutrients, preferably DMEM/F12, fetal bovine serum, basic fibroblast growth factor (bFGF), 2-mercaptoethanol, or any combination thereof. Any compound mentioned in the examples can be included in the kit.

It is contemplated that any method or product described herein can be implemented with respect to any other method or product described herein and that different embodiments may be combined.

It is contemplated that any embodiment discussed herein can be implemented with respect to any method or product of the invention, and vice versa. Any embodiment discussed with respect to a particular condition can be applied or implemented with respect to a different condition. Furthermore, compositions and kits of the invention can be used to achieve methods of the invention.

"Comprising" is understood as an open ended term, i.e. allowing further components or steps of substance. "Consisting of" is understood of a closed term without any further components or steps of substance.

Throughout this application, the term "about" may be used to indicate that a value includes the standard deviation of error for the device or method being employed to determine the value or in a set value may refer to ± 10%.

The present invention is further illustrated by the following figures and examples, without being restricted to these embodiments of the invention.

### Figures:

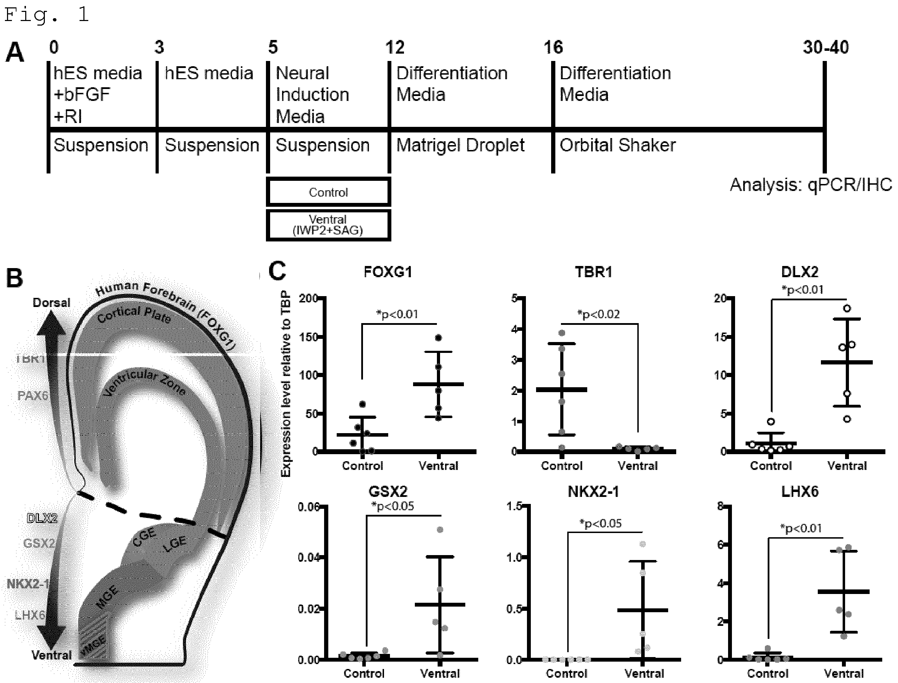

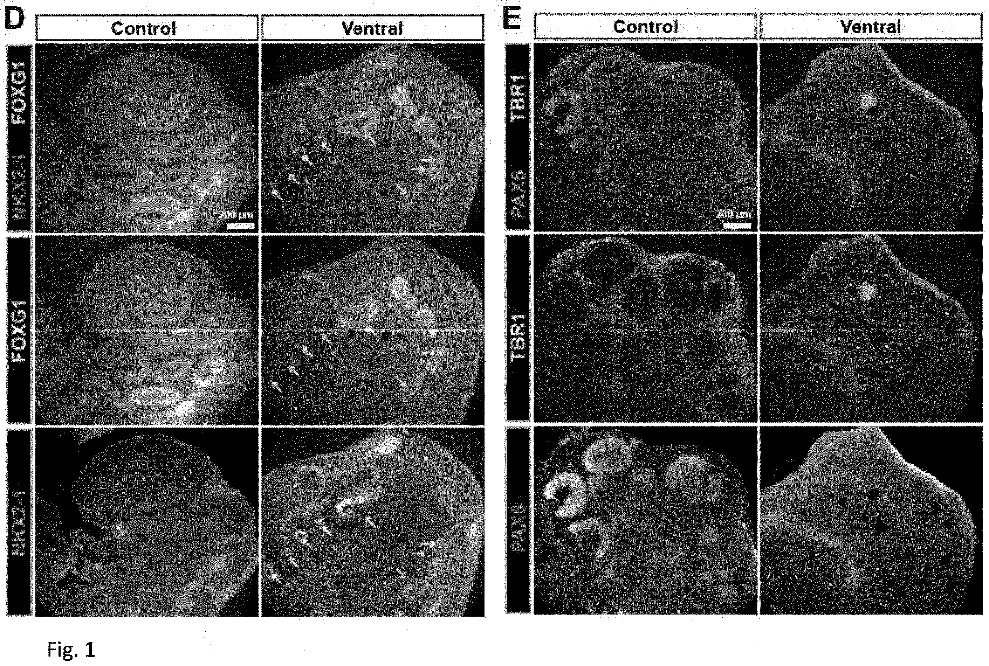

**Figure 1****.** Ventral drug-treatment produces ventral-forebrain containing cerebral organoids. (A) Schematic of cerebral organoid protocol with ventral drug-patterning application (2.5µM IWP2 and 100nM SAG) during the neural induction step. (B) Schematic of a human coronal brain slice indicating the regional expression of the patterning markers used for qPCR/IHC analysis. (C) qPCR analysis of the expression of different brain regional markers showing an increase in ventral and decrease in dorsal forebrain identity in ventral cerebral organoids. Values are plotted as relative expression level (2^{-ΔCt}) to the reference gene TBP. Each data point corresponds to a pooled batch of 8-10 organoids. Data is represented as mean±SD, and statistical significance was tested using the student's t-test (df=9) for control (n=6 batches) versus IWP2+SAG (n=5 batches) treatment. (D-E) Widefield images of immunostaining analysis of control and ventral cerebral organoids indicating the ventral-forebrain identity of ventral organoids (D), and the dorsal-forebrain identity of control organoids (E). Scale bars are 200µm.

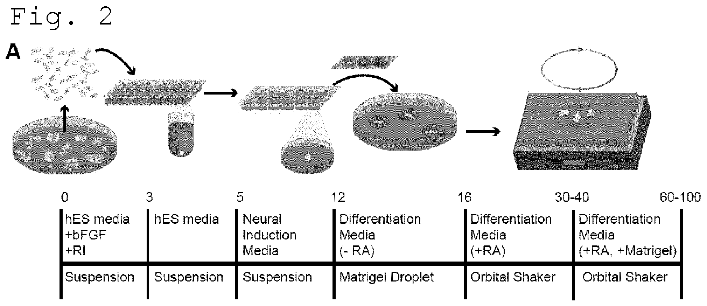

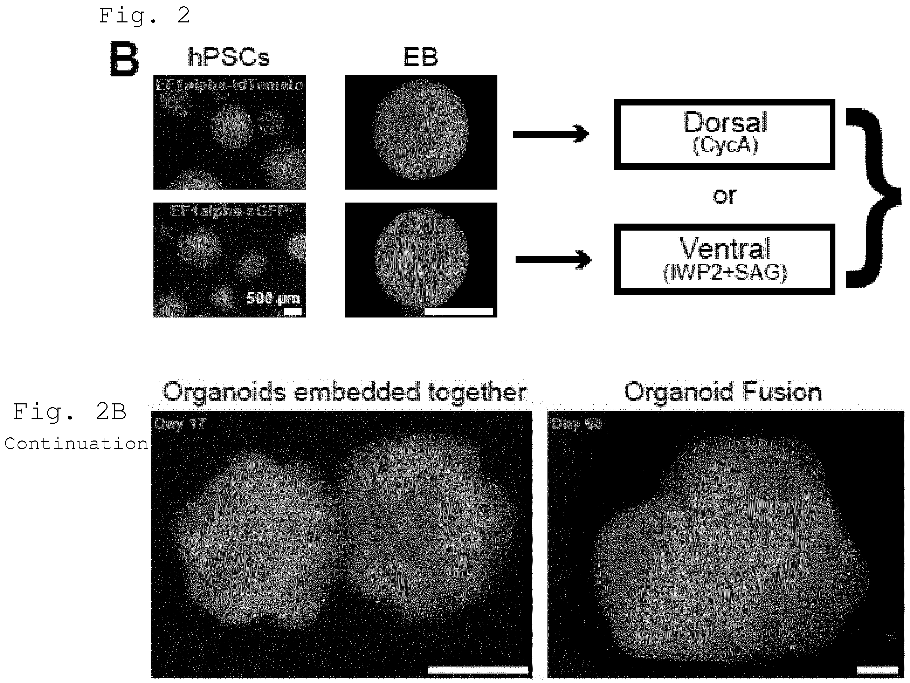

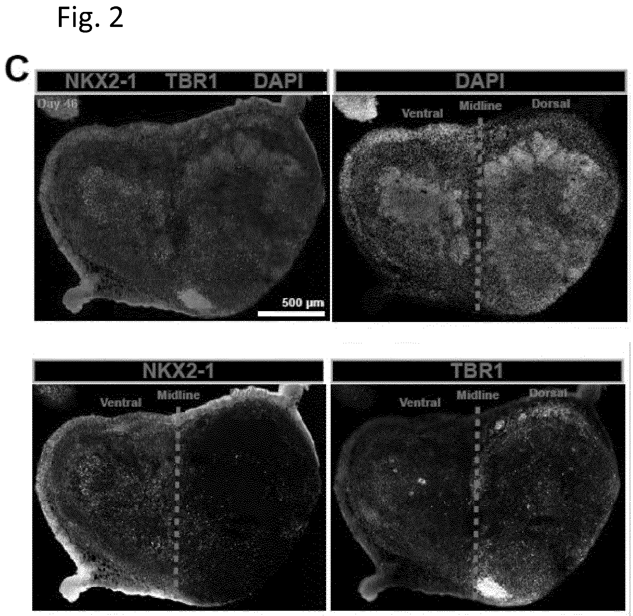

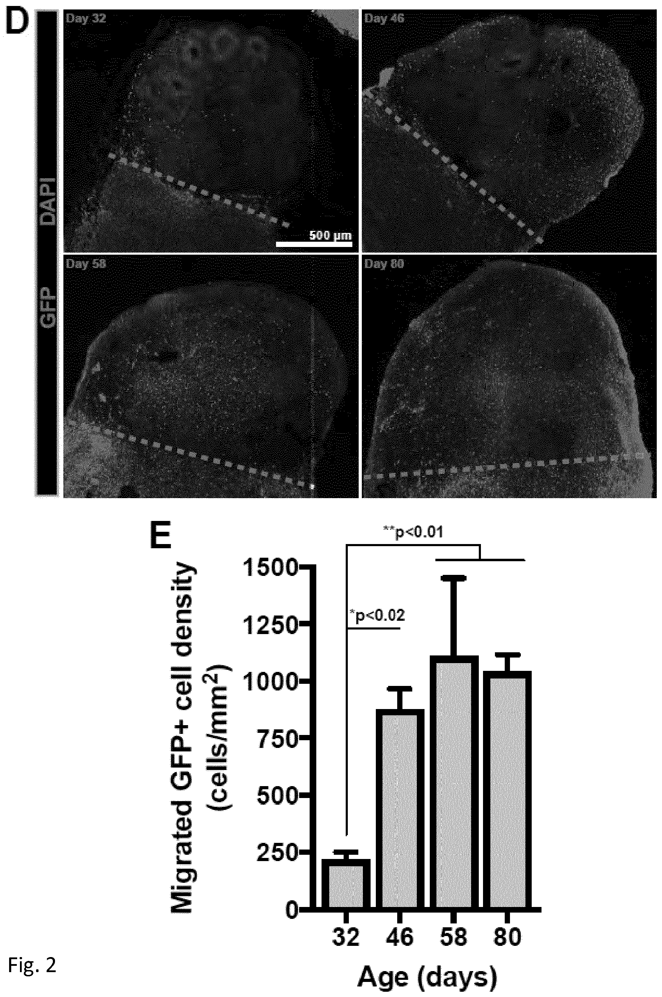

**Figure 2****.** Fusion of cerebral organoids allows cell migration between ventral and dorsal forebrain tissue. (A) The experimental outline of the cerebral organoid fusion co-culture method. (B) Representative widefield images at different stages throughout the organoid fusion procedure. (C) Tile-scan image of an immunostained cryosection of a ventral::dorsal organoid fusion indicates the combination of ventral (NKX2-1⁺) and dorsal (TBR1⁺) regions. (D) Immunostained ventral::dorsal organoid fusion cryosections from organoids of different ages shows the time course of GFP⁺ cells migrating from ventral into dorsal tissue. (E) The GFP⁺ cell density in dorsal tissue was quantified in sections from 32 (n=3 organoids) 46 (n=3), 58 (n=4), and 80 (n=4) day-old organoids. The data is presented as mean±SD and statistical significance tested using the one-way ANOVA [F(3,10)=12.59, p=0.0010] with posthoc Tukey's test for between group comparisons. Scale bars are 500µm.

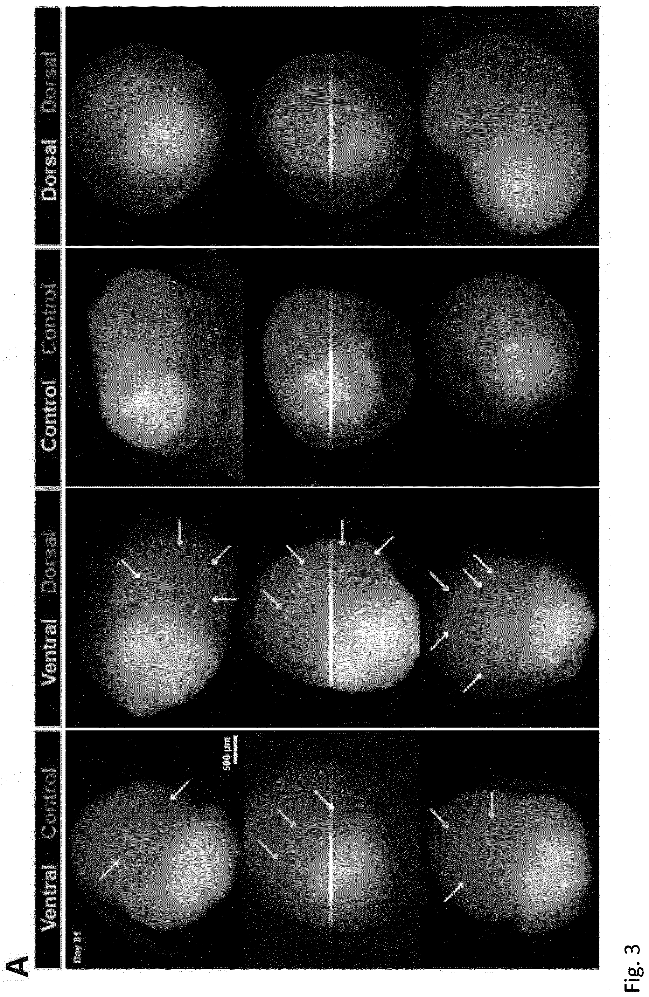

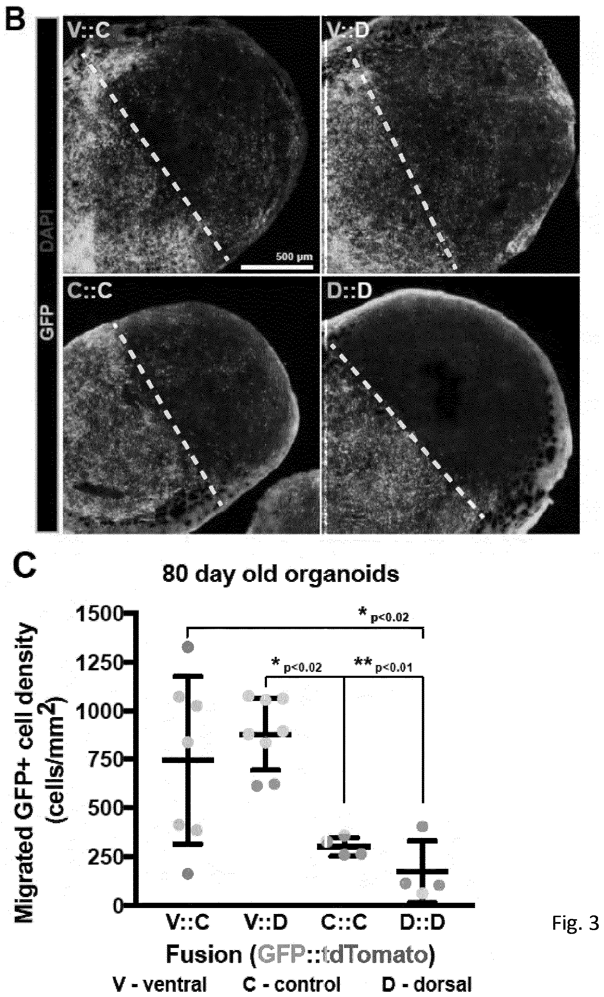

**Figure 3****.** Mixing the tissue components of cerebral organoid fusions indicates the most robust migration from ventral into dorsal regions. (A) Cerebral organoid fusions were created containing different combinations of ventral (V), control (C) or dorsal (D) treated tissue. The components were labeled with either GFP (green) or tdTomato (red). (A) Whole mount images of ~80 day old organoid fusions show the emergence of GFP⁺ spots (arrows) in tdTomato⁺ tissue in ventral-control and ventral-dorsal organoid fusions. (B) Tile-scan confocal images of immunostained mixed organoid fusion cyrosections shows migration of GFP⁺ cells across the midline (dashed line) into the GFP- organoid. (C) Quantification of GFP⁺ cell density in GFP⁻ tissue from tissue sections. Each data point corresponds to an individual organoid, and the data is represented as mean±SD with statistical significance tested using one-way ANOVA [F(3,19)=8.214, p=0.0010] with posthoc Tukey's test for between group comparisons. The ventral::control (n=7 organoids) and ventral::dorsal (n=8) fusions show the most migration of GFP⁺ cells compared to control::control (n=4) and dorsal::dorsal (n=4) fusions. Scale bars are 500µm.

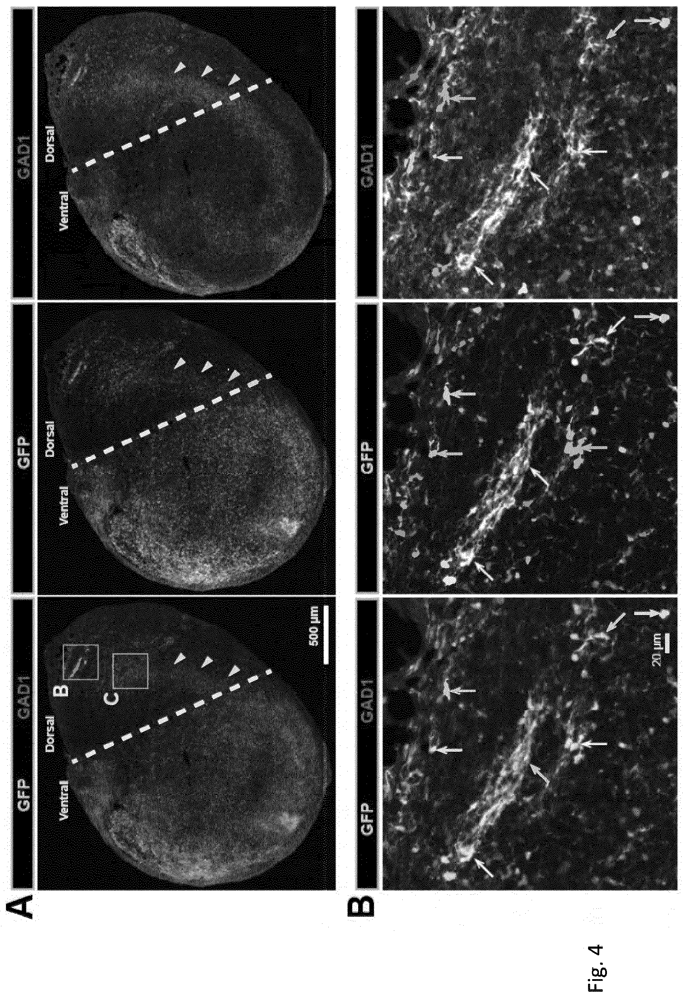

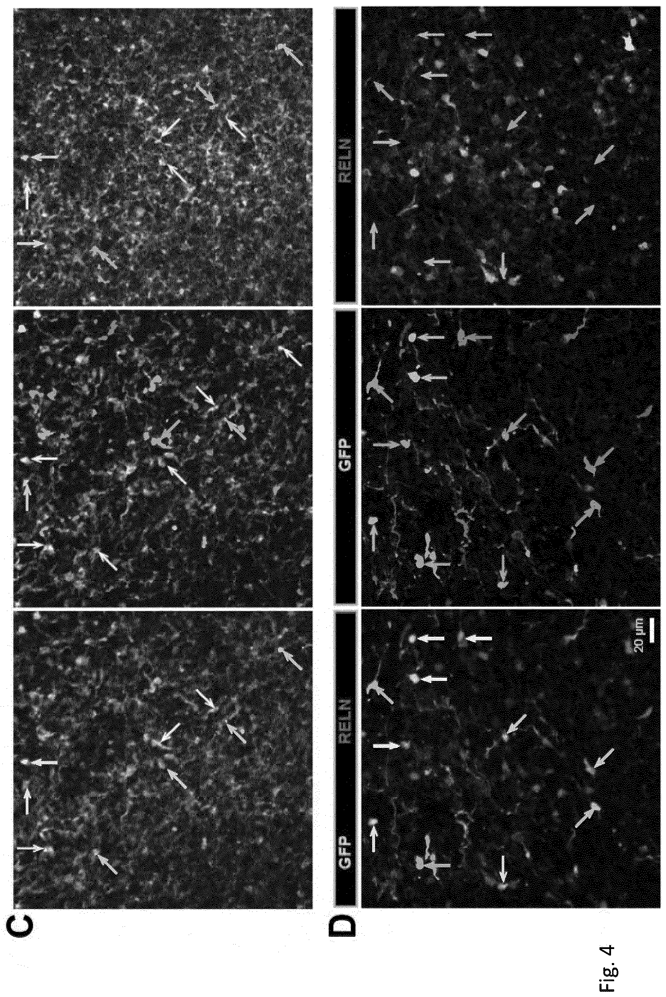

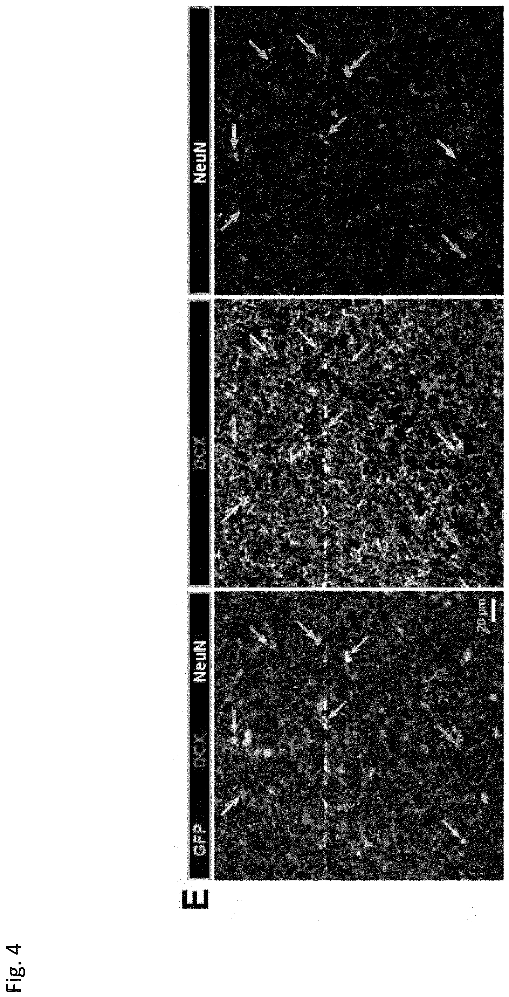

**Figure 4****.** GABAergic interneurons migrate between fused dorsal-ventral cerebral organoids. (A) A whole organoid confocal tile-scan image of an immunostained 80-day old ventral::dorsal organoid fusion cryosection. GFP⁺ cells can be observed migrating across the fusion midline (dashed line) from GFP⁺ ventral into GFP⁻ dorsal tissue. The GABAergic marker GAD1 can be observed in a similar pattern as GFP (arrowheads). (B-C) A magnified view of peripheral (B) and internal regions (C) of the organoid fusion in (A). GFP⁺ cells expressing GAD1 can be observed in both regions (arrows). (D) A confocal image of GFP/RELN immunostaining in the dorsal region of an 80-day old ventral::dorsal organoid fusion cryosection showing that migrated GFP⁺ cells (arrows) do not express RELN. (E) A confocal image of GFP/DCX/NeuN immunostaining in the dorsal region of a 58-day old ventral::dorsal organoid fusion cryosection showing that migrating GFP⁺ cells are DCX⁺ immature neurons (yellow arrows), and some are mature (DCX⁺/NeuN⁺) neurons (blue arrows). Scale bars are (A) 500µm, (B-E) 20µm.

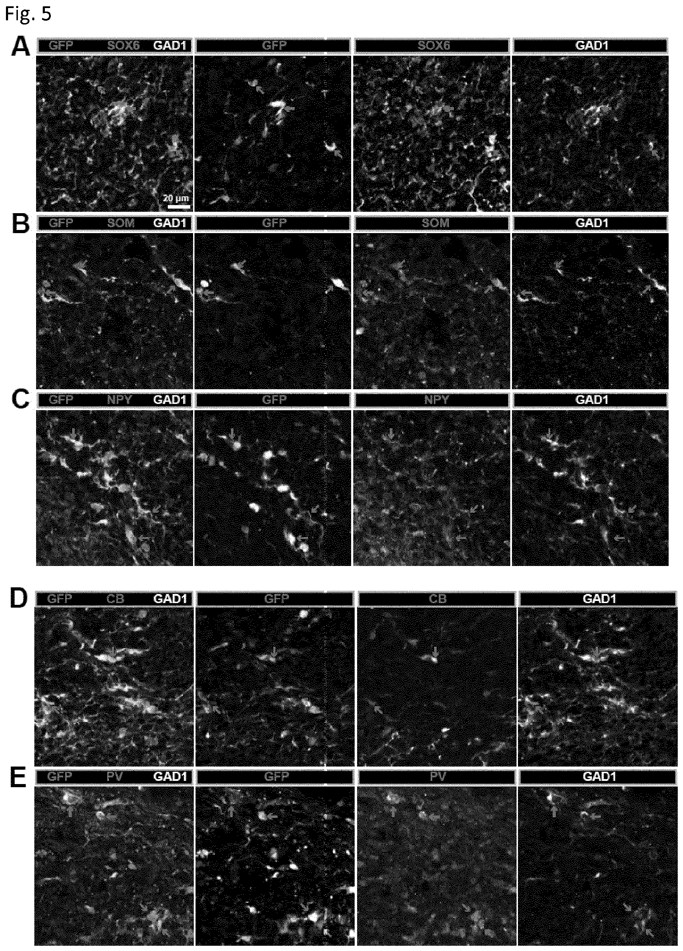

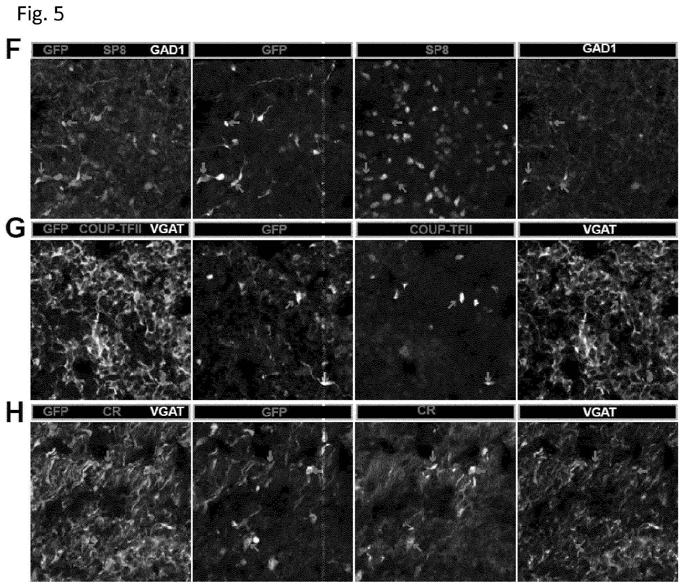

**Figure 5****.** Migrating interneurons in ventral::dorsal cerebral organoid fusions express various interneuron subtype markers. (A-H) Confocal images of immunostaining in dorsal regions of 80-day old ventral::dorsal organoid fusion cryosections. Expression of the GABAergic markers GAD1 or VGAT were used to identify interneurons. Examples of various migrated GFP⁺ interneurons expressing either GAD1 or VGAT were observed expressing the MGE-derived interneuron marker SOX6 (A) or subtype markers SOM (B), NPY (C), CB (D), and PV (E). Migrated GFP⁺ interneurons also expressed the LGE/CGE-derived interneuron markers SP8 (F), COUP-TFII (G), or the subtype marker CR (H). Scale bars are 20µm. Abbreviations: SOM=somatostatin, NPY=neuropeptide Y, CB=calbindin, PV=parvalbumin, CR=calretinin, VGAT=vesicular GABA transporter, GAD1=glutamate decarboxylase 1.

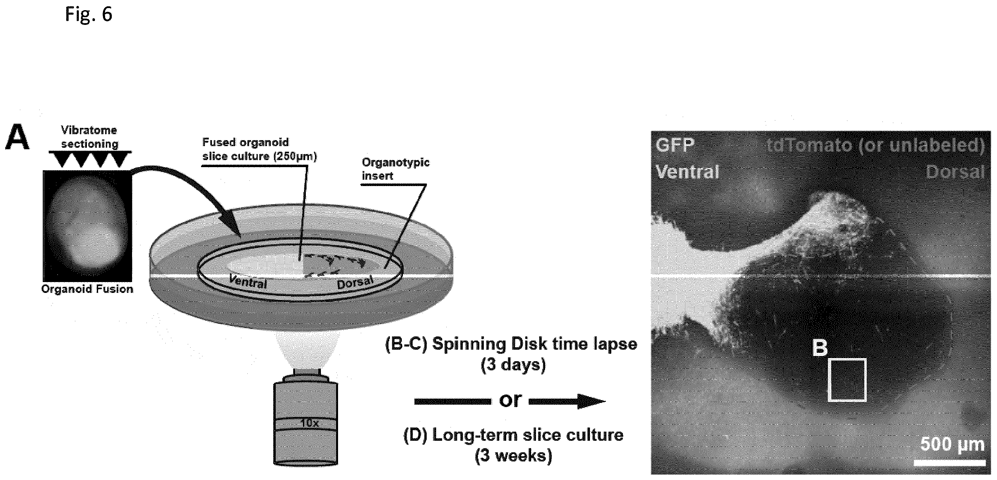

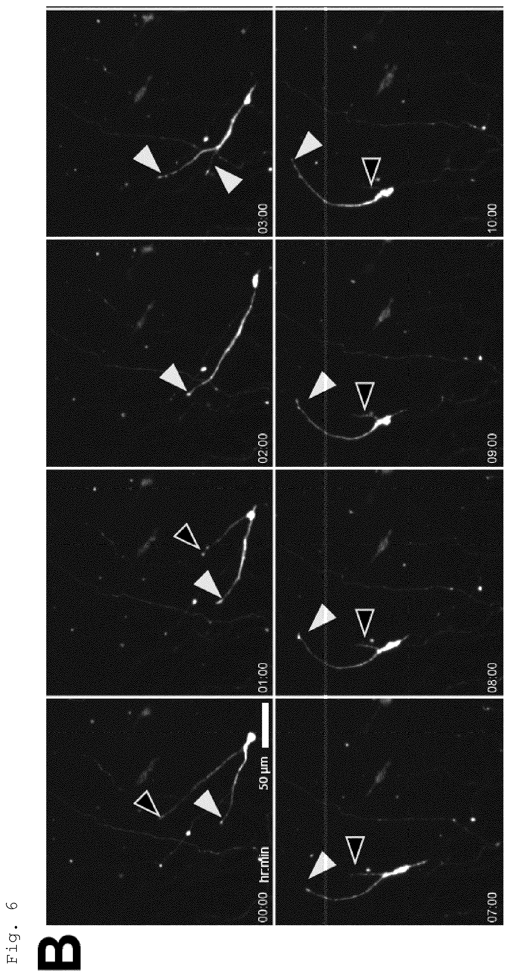

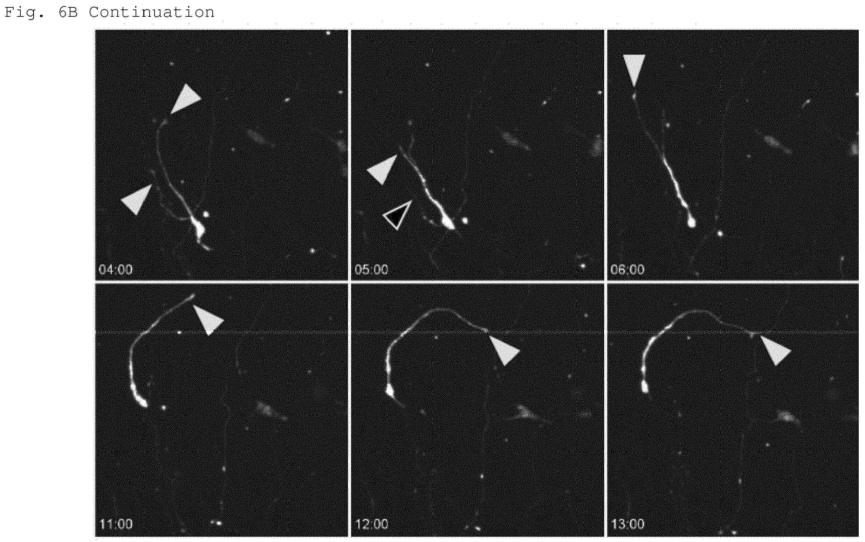

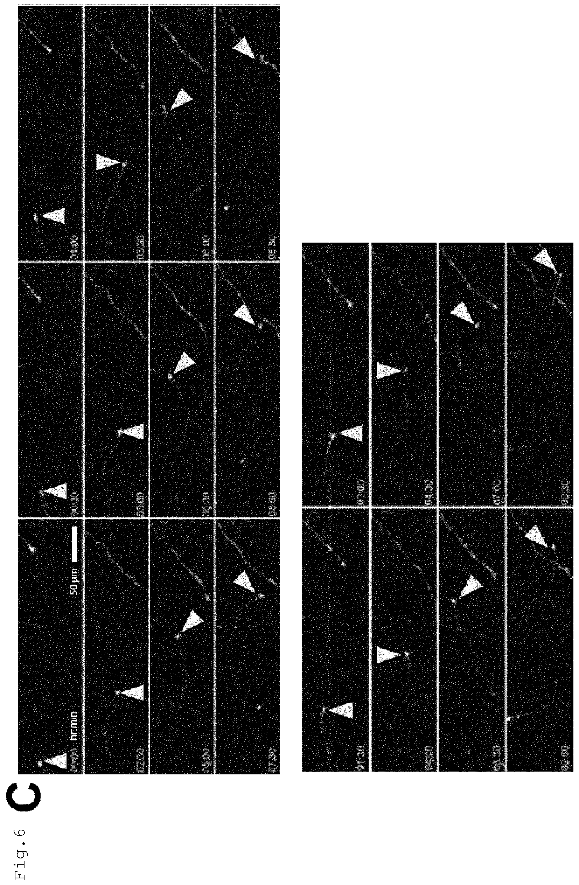

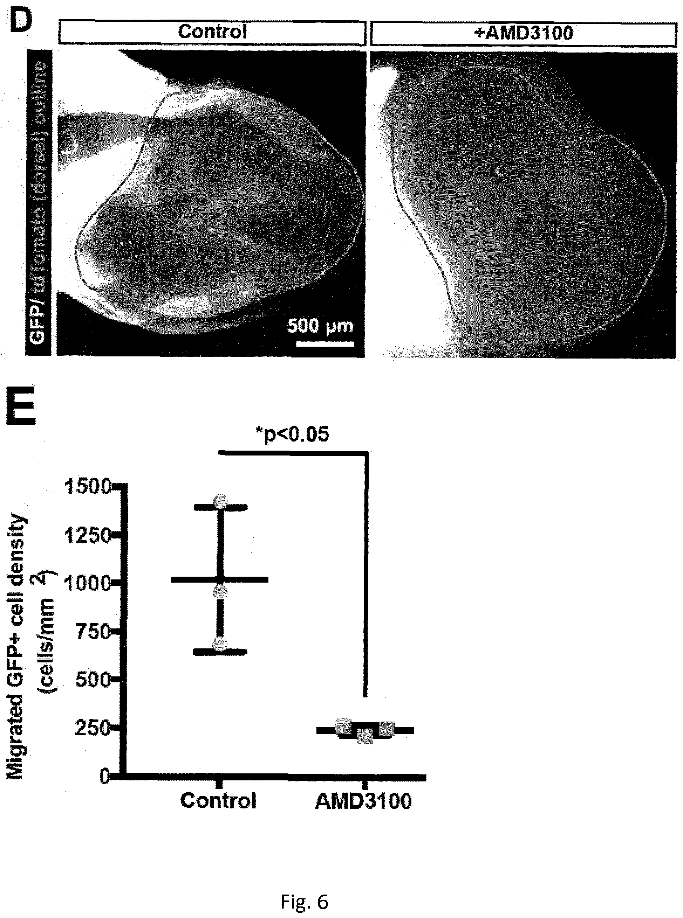

**Figure 6****.** Migrating cells in cerebral organoid fusions exhibit the migratory dynamics of tangentially migrating interneurons and are sensitive to CXCR4 activity. (A) A schematic representation of the organoid fusion slice culture assay for either short-term time-lapse imaging of migratory dynamics, or long-term drug-treatments. A representative tile-scan image of an entire ventral::dorsal organoid fusion slice is shown with the ventral GFP⁺ regions labeled in green and the unlabeled or tdTomato dorsal regions outlined in red. A region containing the migrating cell shown in (B) is noted by a yellow box. (B) Still images from a 3-day time-lapse experiment showing a migrating GFP⁺ cell. The branched leading process exhibits both extending (closed arrowheads) and retracting (open arrowheads) branches as the cell body follows one of the leading processes. (C) An extending neurite (closed arrowhead) with a tuft that appears to be an axon growth cone travels in one direction across the field of view. (D) A widefield image of GFP⁺ cells that migrated into the tdTomato⁺ dorsal region (red outline) from long-term organoid fusion slice cultures that were either untreated (control) or treated with a the CXCR4 inhibitor (AMD3100). (E) Quantification of the migrated GFP⁺ cell density with data represented as mean±SD with statistical significance tested using the student's t-test (df=4) comparing control (n=3 organoids) to AMD3100 (n=3) treatment. Each data point represents a one slice from individual organoids. Fewer migrating cells are observed in AMD3100-treated slice cultures. Scale bars are (A) 500µm, (B-C) 50µm, and (D) 500µm.

### Reference examples

### Example 1: Cell culture

Feeder-dependent human induced pluripotent stem cells (hiPSCs) (Systems Biosciences, cat. no. SC101A-1) were obtained from System Biosciences with pluripotent verification and contamination-free. Feeder-dependent hiPSCs were cultured with irradiated mouse embryonic fibroblast (MEF) feeder cells (MTI-GlobalStem, cat. no. 6001G) on gelatin coated (0.1% gelatin in PBS) 6-well culture plates using human embryonic stem cell (hESC) medium: DMEM/F12 (Invitrogen) containing 20 ng/mL bFGF (produced by IMBA institute Molecular Biology Service core facility), 3.5µL/500mL media of 2-mercaptoethanol, 20% KnockOut Serum (Invitrogen), 1% GlutaMAX (Invitrogen), 1% MEM-NEAA (Sigma), and 3% FBS). Comparative feeder free H9 human embryonic stem cells (hESCs) were obtained from WiCell with verified normal karyotype and contamination-free. Feeder-free hESCs were cultured on hESC-qualified Matrigel (Corning cat. no. 354277) coated 6-well plates using mTeSR1 (Stemcell Technologies). All stem cells were maintained in a 5% CO₂ incubator at 37°C. Standard procedures were used for culturing and splitting hPSCs as explained previously (Lancaster & Knoblich Nat Protoc 9, 2329-2340 (2014)). All cell lines were routinely tested for contamination and confirmed mycoplasma-negative.

### Example 2: Cloning/Molecular biology/Generating hPSC lines

For ubiquitous fluorescent labeling of cells, a reporter construct was inserted into the safe-harbor AAVS1 locus in hPSCs as done previously with TALEN technology (Hockemeyer et al. Nat. Biotechnol. 27, 851-857 (2009)) using the AAVS1 SA-2A-Puro donor vector as a template. A modified backbone was created containing flanking tandem repeats of the core chicken HS4 insulator (2xCHS4). Fluorescent reporter expression cassettes were inserted between the flanking insulator sequences. The following expression constructs were inserted into iPSCs: 1) 2xCHS4-EF1α-eGFP-SV40-2xCHS4, 2) 2xCHS4-EF1α-tdTomato-SV40-2xCHS4. In comparative feeder-free H9 hESCS, a 2xCHS4-CAG-eGFP-WPRE-SV40-2xCHS4 construct was inserted to enhance the GFP expression for time-lapse imaging experiments.

hPSCs were prepared for nucleofection as a single-cell suspension using the same cell dissociation procedures as for making EBs¹¹. The Amaxa nucleofector (Lonza) was used with Stem Cell Kit 1 using 800,000 cells per nucleofection containing 1µg each of the TALEN guides, and 3µg of the donor plasmid DNA following manufacturer guidelines. After nucleofection, 200µL of the total 600 µL nucleofection solution was plated onto a 10 cm cell culture dish. Colonies from single cells were grown for 4 days, and then treated with puromycin (Puro) (Jena Bioscience, cat. no. NU-931-5). For feeder-dependent cells, 1.1 µg/mL puro was applied, and for feeder-free cells 0.7 µg/mL was applied. The puro treatment continued for 5-7 days until the surviving colonies were large enough to be picked manually and transferred into a 24-well plate. When splitting the colonies from a 24-well plate, half of the cells were used for genotyping, while the other half was expanded into 12 and then 6-well formats, and then used for further experiments. For genotyping, DNA was extracted using the QuickExtract solution (EpiCentre), and PCR was performed using the following primers to identify correctly targeted AAVS1 insertions: 1) Puro (*AAVS1_Puro-fwd*: tcccctcttccgatgttgag (SEQ ID NO: 1) and *AAVS1_Puro-rev:* gttcttgcagctcggtgac (SEQ ID NO: 2)), 2) eGFP (*AAVS1*_*eGFP-fwd*: GAACGGCATCAAGGTGAACT (SEQ ID NO: 3) and *AAVS1_eGFP-rev:* cttcttggccacgtaacctg (SEQ ID NO: 4)), and 3) tdTomato (*AAVS1*_*tdtomato-fwd*: ctacaaggtgaagatgcgcg (SEQ ID NO: 5)) and (*AAVS1*_*tdTomato-rev*: tccagcccctcctactctag (SEQ ID NO: 6)). To determine whether the insertion was heterozygous or homozygous, the presence of a WT allele was tested using additional PCR primers: 4) WT (*AAVS1*_*WT-fwd*: tcccctcttccgatgttgag (SEQ ID NO: 7), and *AAVS1*_*WT-rev*: tccagcccctcctactctag (SEQ ID NO: 8)). Cell clones with correctly targeted heteroyzygous or homozygous insertions were archived by freezing with Cell Banker 2 solution (Amsbio, cat. no. 11891) and/or cultured for further experiments.

### Example 3: Cerebral organoid generation and fusion

Cerebral organoids were generated following the previous protocol described in WO2014/090993, Lancaster et al., Nature 501, 373-379 (2013), Lancaster & Knoblich, Science 345, 1247125-1247125 (2014) and Lancaster & Knoblich, Nat Protoc 9, 2329-2340 (2014) with slight modifications. A drug-patterning treatment was applied during the neural induction step of the protocol (~day 5-11) using one of the following treatments: 1) Control, no drugs, 2) Ventral, 2.5µM IWP2 (Sigma, cat. no. I0536) and 100nM SAG (Millipore, cat. no. 566660), 3) Dorsal, 5µM CycA (Calbiochem, cat. no. 239803). Stock drug solutions were created as follows: IWP2 (5mM in DMSO), SAG (1 mM in H₂O), and CycA (5 mM in DMSO). Following embedding in Matrigel (Corning, cat. no. 356235), organoids were grown in 10 cm cell culture dishes containing 25 mL of differentiation medium, and after the first media exchange maintained on an orbital shaker with medium exchange every 5-7 days. After day 40 of the protocol, when organoids begin to grow out of the Matrigel droplet, the differentiation medium was supplemented with 1% Matrigel.

To create organoid fusions, EBs were grown separately and individually patterned using either control, dorsal, or ventral protocols as described above. During Matrigel embedding, two EBs were transferred into the same parafilm well and embedded in a single droplet (~30 µL) of Matrigel. The EBs were gently pushed as close together as possible using a 20 µL pipet tip to ensure the EBs would remain in close proximity within the middle of the solidified Matrigel droplet.

### Example 4: RNA extraction/qPCR

For each drug-patterning treatment group, 8-12 organoids were collected at day 30-40 into 2 mL RNAse-free tubes and chilled on ice throughout the procedure. The organoids were washed 3x in cold PBS, then the Matrigel was dissolved by incubating the organoids in chilled Cell Recovery Solution (Corning, cat. no. 354253) for 1 hr at 4°C. The dissolved Matrigel was removed by rinsing 3x in cold PBS. RNA was extracted using the RNeasy mini kit (Qiagen). cDNA synthesis was performed using 2 µg of total RNA and the Superscript II (Invitrogen) enzyme according to the manufacturer protocols. qPCR reactions were performed using Sybr Green master mix (Promega) on a BioRad 384-well machine (CXF384) using the following reaction protocol: 1) 95°C for 3min, 2) 95 °C for 10s, 3) 62 °C for 10s, 4) 72 °C for 40s, 5) go to 2, 40 cycles, 6) 95°C for 1min, 7) 50 °C for 10s. Quantification was performed in excel by calculating the ΔCt value using TBP as a reference gene. Data is presented as expression level (2^{-ΔCt}) relative to TBP.

### Example 5: Cerebral Organoid Slice Culture and Drug-treatment

Slice cultures were generated by vibratome sectioning organoid fusions. The organoid fusion tissue was embedded in 4% low melt agarose (Biozym, cat. no. 850080), and sectioned in ice-cold PBS (without Ca²⁺/Mg²⁺) to create 200-250 µm sections. The sections were transferred onto Millicell organotypic inserts (Millipore, cat. no. PICM01250) in 6-well cell culture plates. For time-lapse imaging, sections were cultured for 1-2 days before mounting for imaging using a spinning disk confocal microscope (VisiScope). Sections were cut away from the culture insert membrane and inverted onto a glass-bottom dish. The section was immobilized by placing a cell culture insert on top of the section, and attaching it to the dish using vacuum grease. For drug-treatment, long-term slice cultures were initially cultured overnight in differentiation media (+5% FBS). Then the media was exchanged for fresh media (control) or media containing the CXCR4 inhibitor AMD3100 (Sigma, #A5602) and cultured for an additional 3 weeks before tissue fixation and further immunofluorescent processing.

### Example 6: Histology/Cryosectioning/Immunofluorescence

Organoid tissue was collected at the desired age, rinsed 3x in PBS, and fixed in 4% PFA overnight at 4°C. The next day the tissue was rinsed 3x in PBS, and cryoprotected in 30% sucrose in PBS overnight at 4°C. Then the tissue was incubated 2-4 hours in a 1:1 mixture of 30% sucrose/PBS and O.C.T. cryoembedding medium (Sakura, cat. no. 4583). Next, groups of 2-4 organoids were transferred from the sucrose/OCT mixture into a cryomold and filled with O.C.T. The embedded tissue was frozen on dry ice, then placed in -80°C for long term storage until cryostat sectioning. Frozen organoid tissue was sliced into 20 µm sections using a cryostat (Leica), and collected on superfrost Ultra Plus slides. Tissue sections were arranged such that every 10^{th} slice was collected sequentially until each slide contained 8-10 sections per block of tissue. The sections were dried overnight, and then used for immunofluorescent labeling, or stored at -20°C until ready to stain.

Immunofluorescence was performed on tissue sections directly on slides. The O.C.T. was removed by washing 10 minutes in PBS using a slide rack (Sigma, Wash-N-Dry). The sections were post-fixed directly on the slides using 4% PFA for 10 minutes at RT followed by washing 3x10 minutes in PBS. The tissue area was outlined using a hydrophobic PAP pen. Permeabilization/blocking was performed using 5% BSA/0.3% TX100 in PBS with 0.05% sodium azide and incubated 30 minutes at room temperature (RT) within a dark humidified slide box. Primary antibodies (a table of used primary and secondary antibodies can be found at the end of the method section) were added at the desired dilutions in antibody solution (5% BSA, 0.1% TX100 in PBS with 0.05% sodium azide) and incubated overnight at 4°C. Slides were rinsed 3x in PBS and then washed 3x10 minutes in PBST at RT on an orbital shaker. Secondary antibodies were added at 1:500 in antibody solution and incubated at RT for 2 hours. DAPI solution (2 µg/mL) was added for 5 minutes, then slides were washed as done after primary antibody application, with the final washing using PBS. Coverslips were mounted using DAKO mounting medium (Agilent Pathology Solutions, cat. no. S3023) and allowed to harden overnight. Slides were then stored at 4°C until imaging, or at -20°C for long-term storage.

For long-term slice cultures, slices grown on cell culture inserts were rinsed in PBS, and then fixed in 4% PFA for 2 days at 4°C. The sections were washed 3x10 minutes in PBS, then perme-abilized/blocked as performed for cryosections on slides. Primary and secondary antibody incubations were performed overnight at 4°C, followed by washing 3x10 minutes in PBS after each step. The sections were mounted in Vectashield containing DAPI (Vector Labs).

### Example 7: Imaging/Microscopy

Fluorescent cell culture imaging was performed using a Zeiss Axio Vert.A1 widefield microscope and an Axiocam ERc 5s camera (Zeiss, Zeiss GmbH) using Zeiss Plan-Neofluar 2.5x 0.085 and Zeiss LD A-Plan 10x 0.25 Ph1 objectives. Both tdtomato and GFP channels were recorded separately and subsequently pseudocolored and merged using the Fiji package of ImageJ.

Widefield imaging of IHC stainings and long-term slice cultures was performed using an Axio Imager Z2 (Zeiss, Zeiss GmbH) using a Sola SM2 illumination source, (5x 0.15 plan-neofluar, 10x 0.3 plan-neofluar, 20x 0.5 plan-neofluar) objective and a Hamamatsu Orca Flash 4 camera. Filters used were Ex360/40nm Em 445/50nm, Ex480/40nm Em 535/50nm and Ex560/55nm Em 645/75nm.

Confocal imaging was performed using a Zeiss LSM700 AxioImager with a 20x 0.8 plan-apochromat dry objective. Lasers of 405nm (5mW), 488nm (10mW), 555nm (10mW) and 639nm (5mW) together with, corresponding to wavelength, filters SP490, SP555, SP640 and LP490, LP560, LP640 were used for recording. For whole organoid tile scans the XY Scanning Stage and the Zeiss Zen implemented stitching algorithm was used. For colocalization of markers, Z-scans at 20x were performed.

Imaging of GFP stained slides used for cell density counting, and time-lapse migration were acquired using a Yokogawa W1 spinning disk confocal microscope (VisiScope, Visitron Systems GmbH, Puchheim, Germany) controlled with the Visitron VisiView software and mounted on the Eclipse Ti-E microscope (Nikon, Nikon Instruments BV) using ex 488 laser and em filter 525/50. Measurements were recorded using the 10x 0.45 CFI plan Apo lambda (Nikon, Nikon Instruments BV) objective with the sCMOS camera PCO edge 4.2m. Whole IHC slice imaging for cell counting was performed using the tile scan acquisition function. Tile scans were then stitched using the Grid/Collection stitching plugin ⁵¹ in Fiji. For time lapse movies, tile scan Z-stacks were recorded. After stitching as performed for cell counting, a maximum Z-projection was created, and the Z-projection time stacks used for global visualization of cell migration. Cropped regions were created using Fiji and saved as uncompressed AVI files. The AVI files were converted to the mp4 format using Handbrake software to generate the supplemental movies.

For generation of figures and presenting images, the "crop" function, and changing the max/min levels using the "Brightness/Contrast" function of Fiji were used.

### Example 8: Cell density quantification

The number of GFP⁺ cells migrating into GFP⁻ target regions of cerebral organoid fusions was counted manually using the "Cell Counter" plugin of Fiji. The GFP⁻ area in organoid fusions was outlined with the ROI tool and the area calculated using the "measure" function. The cell counts were divided by the area to determine the density of migrated GFP⁺ cells. For the migration time course (Figure 2E,F) and organoid fusion mixing (Figure 3B,C) experiments, confocal tile scan images of tissue cryosections were used for quantification. For the long-term slice culture experiment (Figure 6D,E), a wide-field 5x image containing the GFP⁻ region was used for counting.

### Example 9: Statistics

All graphs and statistical analyses were generated using Prism 7 (GraphPad). When comparing only two groups (Figure 1C, 6E) an unpaired two-tailed Student's t-test was performed, for the remaining data comparing multiple groups a one-way ANOVA with posthoc Tukey's test was used (Figure 2F, 3C). Samples were tested for normality prior to testing statistical significance. No statistical methods were used to predetermine the sample size. Sample sizes for experiments were estimated based on previous experience with a similar setup that showed significance. Experiments were not randomized and investigator was not blinded.

### Primary Antibodies

| **Species** | **Antigen** | **Company** | **Catalog no.** | **Dilution** |
|---|---|---|---|---|
| Rb | FoxG1 | Abcam | ab18259 | 1:200 |
| Rb | TBR1 | Abcam | ab31940 | 1:300 |
| Rb | PV | Swant | PV27 | 1:1000 |
| Rb | Calbindin | Swant | CD38a D-28K | 1:1000 |
| Ms | Calretinin | Swant | ? | 1: 1000 |
| Ms | GAD1/GAD67 | Millipore | MAB5406 | 1:800 |
| Rb | VGAT | Synaptic Systems | 131 013 | 1:500 |
| Chk | GFP | Abcam | ab13970 | 1:1000 |
| Rb | NPY | Abcam | ab30914 | 1: 1000 |
| Ms | Reelin | Millipore | MAB5366 | 1:200 |
| Rb | SOX6 | Abcam | ab30455 | 1:500 |
| Ms | COUP-TFII | Perseus Proteomics | | 1:300 |
| Gt | Sp8 | Santa Cruz | sc-104661 | 1:300 |
| Rb | VIP | Immunostar | 20077 | 1:750 |
| Gt | DCX | Santa Cruz | sc-8066 | 1:200 |
| Ms | NeuN | Millipore | MAB377 | 1:500 |
| Rat | SOM | Millipore | MAB354 | 1:200 |
| Ms | Nkx2.1 | Dako | M3575 | 1:50 |
| Ms | PAX6 | DSHB | PAX6-s | 1:200 |
| Rb | DsRed/tdtomato | Clontech | 632496 | 1:250 |

### Secondary Antibodies

| **Host** | **Recognizes** | **Fluophore** | **Company** | **Catalog no.** | **Dilution** |
|---|---|---|---|---|---|
| Donkey | rabbit | Alexa Fluor 568 | Invitrogen | A10042 | 1:500 |
| Donkey | rabbit | Alexa Fluor 647 | Invitrogen | A31573 | 1:500 |
| Donkey | mouse IgG | Alexa Fluor 568 | Invitrogen | A10036 | 1:500 |
| Donkey | mouse IgG | Alexa Fluor 647 | Invitrogen | A31571 | 1:500 |
| Donkey | chicken | Alexa Fluor 488 | Jackson Immuno | 703-605-155 | 1:500 |
| Donkey | goat | Alexa flour 647 | Invitrogen | A21447 | 1:500 |
| Donkey | rat | Alexa Fluor 647 | Jackson Immuno | 712-605-150 | 1:500 |

### qPCR Primers

| **Gene** | **Primer 1 sequence** | **Primer 2 sequence** |
|---|---|---|
| DLX2 | ACGTCCCTTACTCCGCCAAG (SEQ ID NO: 9) | AGTAGATGGTGCGGGGTTTCC (SEQ ID NO: 10) |
| FOXG1 | TGGCCCATGTCGCCCTTCCT (SEQ ID NO: 11) | GCCGACGTGGTGCCGTTGTA (SEQ ID NO: 12) |
| GSX2 | CACCGCCACCACCTACAAC (SEQ ID NO: 13) | CAGGAGTTGCGTGCTAGTGA (SEQ ID NO: 14) |
| LHX6 | CCGTCTGCAGGCAAGAACAT (SEQ ID NO: 15) | GACACACGGAGCACTCGAG (SEQ ID NO: 16) |
| NKX2-1 | GCCGTACCAGGACACCATG (SEQ ID NO: 17) | ATGTTCTTGCTCACGTCCCC (SEQ ID NO: 18) |
| TBP | GGGCACCACTCCACTGTATC (SEQ ID NO: 19) | CGAAGTGCAATGGTCTTTAGG (SEQ ID NO: 20) |
| TBR1 | CTCAGTTCATCGCCGTCACC (SEQ ID NO: 21) | AGCCGGTGTAGATCGTGTCATA (SEQ ID NO: 22) |

### Example 10: Differentiation by drug-patterning of cerebral organoid tissues enhances the production of ventral forebrain identity.

The cerebral organoid method (WO2014/090993, Lancaster et al., both Lancaster & Knoblich, all *supra*) is capable of producing many different brain regions, including dorsal and ventral forebrain. As it relies on intrinsic patterning, however, the production of some regions is variable and infrequent, especially more ventral (NKX2-1+) interneuron progenitor regions. To increase the consistency and yield of ventral-forebrain interneuron progenitor regions, we included a ventral drug-treatment (Figure 1A). We utilized a combination of WNT-inhibition and enhanced SHH signaling to promote a rostro-ventral forebrain identity. qPCR analysis of the ventral organoids revealed a significant increase in expression of the forebrain marker FOXG1 (Figure 1B,C). The dorsal forebrain marker TBR1 became undetectable, while the ventral forebrain marker DLX2 was dramatically increased in ventral organoids compared to control organoids (Figure 1B,C). To further confirm the successful ventralization of cerebral organoid tissue, we examined the expression of specific markers of ventral forebrain ganglionic eminence (GE) subregions that produce interneurons of different subtypes. GSX2 is expressed in the dorsal-lateral GE (LGE) and caudal GE (CGE), NKX2-1 is expressed in the ventral-medial GE (MGE), while LHX6 is expressed in a subregion of the MGE producing more ventral-derived MGE (vMGE) interneurons (Figure 1B). GSX2 was expressed in control organoids, but further increased in ventral organoids (Figure 1C). Expression of NKX2-1 and LHX6 was undetectable in control organoids, but largely increased in ventral organoids (Figure 1C). Finally, immunostaining confirmed the qPCR results indicating widespread expression of FOXG1 in control and ventral organoids, but only ventral organoids expressed the ventral forebrain marker NKX2-1 (Figure 1D). Intriguingly, control organoids widely expressed dorsal forebrain markers for both progenitors (PAX6) and early born neurons (TBR1) (Figure 1E). In contrast, ventral organoids contained only small regions of PAX6⁺ or TBR1⁺ tissue (Figure 1E). Therefore, these results confirm the successful production of ventral cerebral organoids at the expense of dorsal tissue upon ventral drug-treatment.

### Example 11: Fused cerebral organoid tissues recapitulate a continuous dorsal-ventral forebrain axis and long-distance cell migration.

To recreate the complete dorsal-ventral identity axis in a single tissue, we developed an organoid tissue co-culture method, which we termed organoid "fusion". Control organoids produced mostly dorsal forebrain tissue (Figure 1C,E). However, inhibition of SHH activity with the smoothened receptor inhibitor cyclopamine A (CycA) can enhance dorsal forebrain identity in 2D neuronal differentiation from hPSCs. Therefore, to support dorsal identity, organoids were treated with CycA during the neural induction step of the cerebral organoid protocol (Figure 2A). In this approach, embryoid bodies (EBs) are individually differentiated and patterned into either dorsal (CycA) or ventral (IWP2+SAG) forebrain organoids (Figure 2A,B). After differentiation treatments, a ventral and a dorsal EB are embedded together within a single Matrigel droplet (Figure 2A), and over time the organoids grow together and become fused (Figure 2B). Immunostaining of fused ventral::dorsal organoids revealed the production of a continuous tissue where one side was highly positive for the ventral marker NKX2-1, while the opposite side was positive for the dorsal marker TBR1 (Figure 2C). Therefore, the organoid fusion method allows dorsal and ventral forebrain regions to be juxtaposed in an arrangement similar to that occurring during brain development.

To test whether cells could migrate between the fused organoids we used cell lines containing a ubiquitous GFP reporter to create ventral/GFP⁺::dorsal organoid fusions. Immunofluorescent analysis showed many GFP⁺ cells from the ventral organoid within the GFP⁻ dorsal organoid (Figure 2D). Migrating cells were observed in small numbers around day 30 (Figure 2D,E). Their density drastically increased from day 30 to 46, but we did not observe a significant increase from day 46 to 80 (Figure 2E). Since the organoids increase in size with age, the absolute numbers of migrated cells must increase over time to maintain a similar density. Moreover, the cells appeared more dispersed throughout the dorsal regions in 80 day old organoids (Figure 2D). Therefore, based on these results, we focused our future analysis on organoids older than 60 days old.

### Example 12: Mixing the tissue components of cerebral organoid bi-differentiated fusions indicates directed ventral-to-dorsal cortical cell migration.

During *in vivo* brain development, GABAergic interneurons originate in ventral forebrain progenitor regions before they migrate to their dorsal forebrain target. To test whether this directionality is recapitulated in fused organoids, we varied the identities of their individual tissue components (Figure 3). We consistently labeled one organoid with GFP and the other with tdTomato, and delineated the fusions in an origin::target (GFP::tdTomato) arrangement. Using this paradigm, we analyzed the number of migrating cells when differentially controlling the dorsal/ventral identity of the origin and target regions of cerebral organoid fusions. Whole-mount imaging of ventral::control and ventral::dorsal fusions indicated the occurrence of GFP⁺ spots within the tdTomato⁺ tissue (Figure 3A). Conversely, the spots were rarely observed in control::control or dorsal::dorsal fusions (Figure 3A). This difference was even more striking when analyzing immunostained cyrosections of organoid fusion tissue. The highest amount of migrating cells occurred in ventral::control and ventral::dorsal fusions, while the least migration occurred in dorsal::dorsal fusions (Figure 3B,C). Although the average density of migrating cells in ventral::control fusions was not significantly different than ventral::dorsal fusions, the ventral::dorsal fusions more consistently contained a high amount of migrating cells (Figure 3C). These data confirm our initial observation of directionally biased migration from ventral into dorsal organoid tissue, and strongly suggests that the migration between fused organoids resembles interneuron migration. Finally, this experiment shows that ventral::dorsal fused organoids produce the most robust migration between organoid tissues.

### Example 13: GABAergic interneurons migrate between fused cerebral organoid tissues.

Since example 12 suggested that the migration in ventral::dorsal organoid fusions resembles interneuron migration, we first tested whether the migrating cells were GABAergic by examining whether the migrating GFP⁺ cells expressed GAD1, one of the key enzymes for the synthesis of GABA. Immunostaining revealed that the GFP⁺ cells that had migrated into the target organoid broadly expressed GAD1 (Figure 4A-C). Strikingly, when visualizing the entire organoid, GAD1 was expressed in a similar pattern as the GFP⁺ migrating cells (Figure 4A). Additionally, the expression of GAD1 appeared stronger in regions near the edge of the organoid (Figure 4B), and farther away from the origin of the migrating cells. Therefore, interneurons can migrate from ventral into dorsal regions within organoid fusions.

Another major population of tangentially migrating cells in the developing human brain are the non-GABAergic Cajal-Retzius cells. These cells are identified by the expression of Reelin (RELN). Therefore, we tested whether the migratory GFP⁺ cells in organoid fusions also express RELN. Surprisingly, we observed an apparent lack of expression of RELN by GFP⁺ cells (Figure 4D), despite the substantial presence of GFP⁻ , RELN⁺ cells in the dorsal target region of organoid fusions. Thus, our data indicate that the migratory GFP+ cells in organoid fusions are not Cajal-Retzius cells, and in combination with our previous results strengthens their identity as GABAergic interneurons.

Finally, we tested whether the migrating cells could produce mature neurons by analyzing their expression of the immature migrating neuron marker DCX, and/or the mature neuronal marker NeuN. We observed that the GFP⁺ cells expressed DCX (Figure 4E), confirming their neuronal identity, and a subset also expressed NeuN (Figure 4E). This finding indicates that the migrating GFP⁺ cells are migratory neurons (DCX⁺), but also that the migratory neurons can become mature neurons (DCX⁺/NeuN⁺). Taken together, our results show that interneurons can migrate between fused ventral-dorsal organoids, and can become mature neurons.

### Example 14: Migrating interneurons produce various LGE/CGE and MGE-derived cortical interneuron subtypes in fused cerebral organoid tissues.

Since the migrating GFP⁺ cells can become mature neurons (Figure 4E), and appear to be predominantly GABAergic interneurons (Figure 4A,B) we next tested which interneuron subtypes are produced. Interneurons are particularly heterogeneous and multiple molecular markers can be used to identify various subtypes that are generated by distinct progenitor subpopulations within the ventral forebrain. In humans, the majority of interneurons are generated from NKX2-1⁺ regions of the MGE. We tested whether the migrating GFP⁺ cells produce MGE-derived interneuron subtypes. In humans, SOX6 is expressed in the MGE and in immature and mature interneurons emerging from this region. In organoid fusions, we observed multiple SOX6⁺ MGE interneurons among the GFP⁺ migrating cells GFP⁺ cells that were also GAD1⁺ (Figure 5A). Therefore, MGE-derived interneurons can be generated within cerebral organoid fusions. To confirm this finding, we also examined the expression of markers for MGE-derived interneurons. We observed expression of somatostatin (SOM) (Figure 5B), neuropeptide Y (NPY) (Figure 5C) calbindin D-28k (CB) (Figure 5D), and parvalbumin (PV) (Figure 5E) by GFP⁺ migrated interneurons (GAD1+ or VGAT+). Therefore, in organoid fusion tissues, multiple MGE-derived interneuron subtypes can be generated.

The remaining interneurons in the human brain arise from the LGE/CGE. Similar to SOX6 for MGE, the transcription factors COUP-TFII/NR2F2 and SP8 can be used as LGE/CGE fate-mapping markers for cortical interneurons. Both SP8 (Figure 5F) and COUP-TFII (Figure 5G) were expressed by GFP⁺ migrating interneurons (GAD1⁺) in organoid fusions. As confirmation of this result, we also analyzed the expression of markers for LGE/CGE-derived subtypes. Our previous data already indicated a lack of RELN expression by GFP⁺ migrating cells (Figure 4), which in addition to non-interneuron Cajal-Retzius cells is also expressed by subpopulations of GABAergic MGE and CGE-derived interneurons. We also did not observe any vasoactive intestinal peptide (VIP) expressing GFP⁺ interneurons. However, we did observe calretinin (CR) expressing GFP⁺ interneurons (VGAT⁺) in organoid fusions (Figure 5H). Collectively, these results indicate that organoid fusions contain many diverse interneuron subtypes originating from the major ventral forebrain subregions (MGE and LGE/CGE).

### Example 15: Neuronal migration in fused cerebral organoid tissues resembles the tangential migration of cortical interneurons.

The migratory dynamics of tangentially migrating cortical interneurons has been extensively documented using time-lapse imaging experiments. This behavior differs from that of radially migrating cortical cells which move along a radial glia fiber, or that of linearly migrating cells, such as the chain migration observed in LGE-derived olfactory bulb interneurons. To determine whether the behavior of migrating GFP⁺ cells in organoid fusions resembles that of cortical interneurons, we performed time-lapse recordings of migrating GFP⁺ cells in ventral-dorsal organoid fusion slice-cultures (Figure 6A). The GFP⁺ ventral origin region of the organoid fusion could easily be distinguished from the GFP⁻ dorsal target region (Figure 6A). Thus, we could easily visualize the morphology of the sparsely labeled GFP⁺ cells which had migrated into the dorsal organoid fusion region. Over 3 days, we observed both stationary and motile cells (Figure 6B), and surprisingly we also observed dynamic neuronal processes resembling axons that extended long distances (Figure 6C). Strikingly, the migratory cells exhibited dynamics which exactly resembled the dynamics of migrating interneurons in the marginal zone (MZ) and intermediate zone (IZ) of embryonic mouse cortex. Migrating cells contained leading and trailing processes. The leading process was usually branched, and the migratory direction was determined by the branch dynamics. For instance, we observed multiple examples of cells extending a branch in the future direction of travel while retracting a branch that was extended in an alternate direction (Figure 6B). In addition, cells migrated in multiple directions with frequent abrupt changes in direction, which resembles the multidirectional, wandering behavior exhibited by tangentially migrating cortical interneurons within the cortex.

The chemoattractant SDF-1 (CXCL12) and its receptor CXCR4 regulate the tangential migration of interneurons. To test if the migration we observed in cerebral organoid fusions is CXCR4-dependent, we created long-term slice cultures of organoid fusions that we treated with the CXCR4 antagonist, AMD3100. Compared to untreated control slices prepared from the same organoid fusion, the density of migrating GFP⁺ cells into GFP⁻ dorsal regions was significantly reduced upon AMD3100 treatment. Therefore, the cell migration in cerebral organoid fusions depends on CXCR4 activity. Combined with our previous data, this result confirms that the cell migration observed in cerebral organoid is consistent with that of tangentially migrating interneurons.

### Observations:

Migrating GFP⁺ cells within the dorsal region of a ventral/GFP::dorsal organoid fusion were observed. An observed cell migrates in a single direction. The leading process is branched with the different branches dynamically extending and retracting seemingly independent of one another. The trailing process follows as the cell body moves forward, and multiple times a leading process becomes a trailing process. As the cell moves forward, one leading process is extended while the remaining processes retract. Then the whole migratory dynamic cycle is repeated as the cell progresses forward.

A cell exhibiting many changes of direction involving the dynamic extension and retracing of several processes was observed. As the cell body remains static, branches are extended in multiple directions, and then each of the main branches extends additional higher order branches. Finally, a branch is extended in a particular direction followed by the retraction of the other main branch. The cell body is then moved in the direction of the extending branch. The cycle is repeated as the cell decides which direction to migrate.

Multiple migrating cells were observed. 1) Initially a cell is migrating upward. The upward process is retracted as a new leading process is extended downward and becomes branched. The cell migration direction is then changed downward. The bifurcated leading process is dynamic such that one process is extended as the other process is retraced. The cell body then moved toward the extended leading process. Prior to nucleokinesis, a swelling is observed moving from the cell body into the proximal portion of the leading process. Then the cell body is moved in parallel to the swelling, and finally the cell body moves into the swelling. 2) A second cell migrates and changes direction several times. With each change of direction, the trailing process becomes the leading process. The new leading process is extended toward the direction of travel as the trailing process is retracted.

Multiple cells migrating in different directions with extending and retracting processes were observed. Beginning around 45 hrs of observation, one cell migrates and travels rapidly in a constant direction, but at around 70 hours the progress is slowed as the leading process branches.

Multiple migrating cells were observed. Around 6 hours after start of observation, a cell migrates with a branched leading process. At multiple times, 3 branches are observed. As the cell progresses forward, branches are extended in the direction of travel, while other branches are retracted. Around 23 hours the cell changes direction abruptly. This involves an extension of a new process, while the previous leading process is retracted. At around 39 hours, the leading process begins turning then makes a 180-degree turn, and then extends. The cell body then follows the leading process.

The neurites appearing to be axons with an enlarged tuft at the end the processes which resembles that of a growth cone were observed. The processes are highly dynamic, and exhibit extension in single directions, but also abrupt changes in direction.

### Example 16: Cell migration assay

We developed a cell migration assay using fused cerebral organoids. Ventral-dorsal organoid fusions exhibit robust long-distance cell migration resembling that of ventral forebrain-derived cortical inhibitory interneurons. We observed substantially more migration from ventral into dorsal forebrain regions in cerebral organoid fusions. The migrating cells within organoid fusions express GABAergic markers (GAD1/VGAT). The migrated GFP⁺ cells can produce a variety of interneuron subtypes. In addition, the lack of RELN expression by migrating GFP⁺ cells supports a ventral forebrain-derived interneuron identity. The migration dynamics of the GFP⁺ cells in fused organoids resembles the characteristic migratory behavior exhibited by interneurons migrating tangentially within the cortex. Finally, the cell migration in organoid fusions was inhibited by CXCR4 inhibition as observed for tangentially migrating interneurons in developing mouse cortex. In summary, we have recapitulated the migration of human cortical interneurons from ventral in dorsal forebrain regions. Moreover, the observance that these cells exhibit both immature and mature neuronal markers indicates their ability to undergo maturation once arriving in their dorsal cortical forebrain target regions. This represents the exciting opportunity to recreate a developing human cortical circuit containing an enhanced repertoire of cellular diversity than was possible to achieve within the previous singular organoid methods.

An exciting application of this technique is studying human developmental biology and its relationship to neurological diseases. With this in mind while characterizing this assay, we present different experimental paradigms, each of which can be used to investigate various aspects and scientific questions related to neuronal cell migration. For instance, the labeling of the cells within one of the fused organoids with a fluorescent reporter allows the visualization of long-distance neuronal migration. This can even be continuously monitored over time through whole-mount imaging of intact organoid fusions. In addition, the identity of the cells can be easily examined using immunostaining for identification of neuronal subtypes. This is useful because many psychiatric diseases, such as Schizophrenia, are thought to involve selective deficits in specific interneuron subpopulations (Lewis Current Opinion in Neurobiology 26, 22-26 (2014)). A second type of analysis is dissecting the role of specific molecules on neuronal migration to determine if they are acting in a cell-autonomous or cell non-autonomous manner. For instance, using inventive bi-differentiated tissue, organoids from either the origin of migration or the target can be genetically manipulated independently by deriving the origin and target organoids from distinct cell lines containing either mutant or wild-type alleles of a gene of interest.

We also present an organoid fusion slice culture paradigm in which confocal time-lapse imaging can be used to analyze the short-term dynamics of neuronal cell migration. This is an important tool because some molecules such as GABA affect the motility of migrating interneurons, which may produce a subtle phenotype in a long-term migration assay, and therefore instead require a higher time-resolution analysis of the dynamics of migrating cells. In addition, long-term slice cultures can be used to test how different drug-treatments can affect cell migration. This allows drug-screens to determine the effect of many different molecules on cell migration.

Finally, the fused organoid tissues also present unique applications beyond cell migration. As one further example, we observed substantial neurite dynamics with the appearance of axon projections. In addition, although we specifically focused on ventral-dorsal forebrain fusions, the organoid fusion paradigm allows flexibility in the brain regional identities that can be grown together. Combined with the additional brain-region specific organoid protocols (Jo et al. Stem Cell 19, 248-257 (2016); Hockemeyer et al. Nat. Biotechnol. 27, 851-857 (2009); Preibisch et al. Bioinformatics 25, 1463-1465 (2009)), the possible brain circuits that could be modeled using organoid fusions is vast. Therefore, organoid fusion technology greatly enhances the phenotypic analyses possible in cerebral organoids, and the flexibility of the method immensely expands the future development of *in vitro* models of human neurological diseases.

## Claims

1. An *in vitro* method of producing a bi- or multi-differentiated neuronal tissue with at least two neuronal tissue types comprising the steps of providing a first neuronal tissue that is developed to a stage of differentiation of interest; providing a second neuronal tissue that is developed to a stage of differentiation of interest differing from the stage of differentiation of interest of the first neuronal tissue, wherein the volume of the first and/or second tissue is at least 6x10⁶ µm³; placing said first and second neuronal tissue in vicinity sufficient for fusion by growth; allowing the first and second neuronal tissue to grow and fuse to each other; thereby producing a bi- or multi-differentiated neuronal tissue comprising the first and second neuronal tissue with different stages of differentiation.

2. An *in vitro* method of producing a bi- or multi-differentiated neuronal tissue with at least two neuronal tissue types comprising the steps of providing a first neuronal tissue that is developed to a stage of differentiation of interest; providing a second neuronal tissue that is developed to a stage of differentiation of interest differing from the stage of differentiation of interest of the first neuronal tissue; placing said first and second neuronal tissue in vicinity sufficient for fusion by growth; allowing the first and second neuronal tissue to grow and fuse to each other; thereby producing a bi- or multi-differentiated neuronal tissue comprising the first and second neuronal tissue with different stages of differentiation; wherein the first and/or second neuronal tissue is grown in a hydrogel, and/or in extracellular matrix (ECM) or any component thereof selected from collagen, laminin, entactin, and heparin-sulfated proteoglycan or any combination thereof, and/or wherein the tissues during fusion are cultured in a hydrogel, and/or in extracellular matrix (ECM) or any component thereof selected from collagen, laminin, entactin, and heparin-sulfated proteoglycan or any combination thereof.

3. The method of claim 1 or 2, wherein the first and/or the second neuronal tissue, preferably both, are derived from an in-vitro culture of pluripotent stem cells, in particular induced pluripotent stem cells.

4. The method of claim 1 or 2 or 3, comprising developing the first neuronal tissue to a stage of differentiation of interest separate from developing the second neuronal tissue to a stage of differentiation of interest differing.

5. The method of any one of claims 1 to 4, wherein at least the first and/or second neuronal tissue comprises neural progenitor cells and neurons.

6. The method of claim 5, wherein the stages of differentiation of the first neuronal tissue comprises a ventral forebrain progenitor tissue or a rostro-ventral forebrain tissue.

7. The method of claim 5 or 6, wherein the stages of differentiation of the second neuronal tissue comprises dorsal forebrain tissue or neuroectoderm without differentiation to ventral or dorsal forebrain.

8. The method of any one of claims 1 to 7, wherein the steps of placing said first and second neuronal tissue in a vicinity sufficient for fusion by growth and allowing the first and second neuronal tissue to grow and fuse to each other are performed in suspension culture.

9. The method of any one of claims 1 to 8, wherein the bi- or multi-differentiated neuronal tissue is differentiated to develop a neural plate.

10. The method of any one of claims 1 to 9, wherein the first and second neuronal tissue are grown to a size of at least 100 µm, preferably at least 150 µm, especially preferred at least 200 µm.

11. The method of any one of claims 1 to 10, wherein the first and/or second neuronal tissue expresses a detectable marker that is not expressed by the other from the group of the first and second neuronal tissue.

12. A bi- or multi-differentiated neuronal tissue with at least two neuronal tissue types of different stages of differentiation, wherein at least one of the neuronal tissue types comprises ventral neuronal tissue and the at least another neuronal tissue type is substantially non-ventral but containing migrated cells from the ventral neuronal tissue constituting not more than 5% of the cells of the substantially non-ventral tissue and the bi- or multi-differentiated tissue having a size of 100 µm to 10 mm in its longest dimension.

13. The bi- or multi-differentiated neuronal tissue of claim 12, wherein at least one of the neuronal tissue types expresses a detectable marker, preferably a fluorescence marker, not expressed by another tissue type of the bi- or multi-differentiated neuronal tissue.

14. The bi- or multi-differentiated neuronal tissue of any one of claims 12 or 13, in form of a globular body or in form of a tissue slice.

15. A method of testing or screening a candidate compound for influencing differentiation of bi- or multi-differentiated neuronal tissue, comprising contacting cells or a neuronal tissue in a method of any one of claims 1 to 11 with the candidate compound or contacting a neuronal tissue of any one of claims 12 to 14 with the candidate compound and maintaining said contacted tissue in culture, and observing any differentiation al changes in the tissue as compared to said tissue without contacting by said candidate compound.

16. Use of a kit in a method according to any one of claims 1 to 11, wherein the kit comprises a ventral differentiation inducer and a dorsal differentiation inducer, preferably a WNT inhibitor and/or a SHH enhancer as ventral differentiation inducer and preferably a SHH inhibitor as dorsal differentiation inducer.

## Patentansprüche

1. Ein *in vitro* Verfahren zur Herstellung eines bi- oder multidifferenzierten neuronalen Gewebes mit mindestens zwei neuronalen Gewebetypen, umfassend die Schritte vom Bereitstellen eines ersten neuronalen Gewebes, das bis zu einer Differenzierungsstufe von Interesse entwickelt ist; Bereitstellen eines zweiten neuronalen Gewebes, das bis zu einer Differenzierungsstufe von Interesse entwickelt ist, die sich von der Differenzierungsstufe von Interesse des ersten neuronalen Gewebes unterscheidet, wobei das Volumen des ersten und/oder zweiten Gewebes mindestens 6x10⁶ µm³ beträgt; Platzieren des besagten ersten und zweiten neuronalen Gewebes in einer für die Fusion durch Wachstum ausreichenden Nähe; Ermöglichen, dass das erste und zweite neuronale Gewebe wachsen und miteinander fusionieren; wodurch ein bi- oder multi-differenziertes neuronales Gewebes hergestellt wird, das das erste und zweite neuronale Gewebe mit unterschiedlichen Differenzierungsstufen umfasst.

2. Ein *in vitro* Verfahren zur Herstellung eines bi- oder multidifferenzierten neuronalen Gewebes mit mindestens zwei neuronalen Gewebetypen, umfassend die Schritte vom Bereitstellen eines ersten neuronalen Gewebes, das bis zu einer Differenzierungsstufe von Interesse entwickelt ist; Bereitstellen eines zweiten neuronalen Gewebes, das bis zu einer Differenzierungsstufe von Interesse entwickelt ist, die sich von der Differenzierungsstufe von Interesse des ersten neuronalen Gewebes unterscheidet; Platzieren des besagten ersten und zweiten neuronalen Gewebes in einer für die Fusion durch Wachstum ausreichenden Nähe; Ermöglichen, dass das erste und zweite neuronale Gewebe wachsen und miteinander fusionieren; wodurch ein bi- oder multi-differenziertes neuronales Gewebes hergestellt wird, das das erste und zweite neuronale Gewebe mit unterschiedlichen Differenzierungsstufen umfasst; wobei das erste und/oder zweite neuronale Gewebe in einem Hydrogel und/oder in extrazellulärer Matrix (ECM) oder einer beliebigen Komponente davon, ausgewählt aus Kollagen, Laminin, Entactin und Heparan-sulfatiertem Proteoglykan oder einer beliebigen Kombination davon, gezüchtet wird, und/oder wobei die Gewebe während der Fusion in einem Hydrogel und/oder in extrazellulärer Matrix (ECM) oder einer beliebigen Komponente davon, ausgewählt aus Kollagen, Laminin, Entactin und Heparin-sulfatiertem Proteoglykan oder einer beliebigen Kombination davon, kultiviert werden.

3. Das Verfahren nach Anspruch 1 oder 2, wobei das erste und/ oder das zweite neuronale Gewebe, bevorzugt beide, aus einer *in vitro* Kultur von pluripotenten Stammzellen, insbesondere induzierten pluripotenten Stammzellen, abgeleitet sind.

4. Das Verfahren nach Anspruch 1 oder 2 oder 3, umfassend die Entwicklung des ersten neuronalen Gewebes zu einer Differenzierungsstufe von Interesse, getrennt von der Entwicklung des zweiten neuronalen Gewebes zu einer sich unterscheidenden Differenzierungsstufe von Interesse.

5. Das Verfahren nach einem der Ansprüche 1 bis 4, wobei mindestens das erste und/oder zweite neuronale Gewebe neuronale Progenitorzellen und Neuronen umfasst.

6. Das Verfahren nach Anspruch 5, wobei die Differenzierungsstufen des ersten neuronalen Gewebes ein ventrales Vorderhirn-Progenitorgewebe oder ein rostro-ventrales Vorderhirngewebe umfassen.

7. Das Verfahren nach Anspruch 5 oder 6, wobei die Differenzierungsstufen des zweiten neuronalen Gewebes dorsales Vorderhirngewebe oder Neuroektoderm ohne Differenzierung zu ventralem oder dorsalem Vorderhirn umfassen.

8. Das Verfahren nach einem der Ansprüche 1 bis 7, wobei die Schritte vom Platzieren des ersten und zweiten neuronalen Gewebes in einer für die Fusion durch Wachstum ausreichenden Nähe und vom Ermöglichen, dass das erste und zweite neuronale Gewebe wachsen und miteinander fusionieren, in Suspensionskultur durchgeführt werden.

9. Das Verfahren nach einem der Ansprüche 1 bis 8, wobei das bi- oder multi-differenzierte neuronale Gewebe differenziert wird, um eine Neuralplatte zu entwickeln.

10. Das Verfahren nach einem der Ansprüche 1 bis 9, wobei das erste und zweite neuronale Gewebe zu einer Größe von mindestens 100 µm, bevorzugt mindestens 150 µm, besonders bevorzugt mindestens 200 µm gezüchtet werden.

11. Das Verfahren nach einem der Ansprüche 1 bis 10, wobei das erste und/oder zweite neuronale Gewebe einen nachweisbaren Marker exprimiert, der nicht von dem anderen aus der Gruppe des ersten und zweiten neuronalen Gewebes exprimiert wird.

12. Ein bi- oder multi-differenziertes neuronales Gewebe mit mindestens zwei neuronalen Gewebetypen unterschiedlicher Differenzierungsstufen, wobei mindestens einer der neuronalen Gewebetypen ventrales neuronales Gewebe umfasst und mindestens ein ander neuronaler Gewebetyp im Wesentlichen nicht-ventral ist, aber migrierte Zellen aus dem ventralen neuronalen Gewebe enthält, die nicht mehr als 5% der Zellen des im Wesentlichen nicht-ventralen Gewebes ausmachen, und das bi- oder multi-differenzierte Gewebe eine Größe von 100 µm bis 10 mm in seiner längsten Dimension aufweist.

13. Das bi- oder multi-differenzierte neuronale Gewebe nach Anspruch 12, wobei mindestens einer der neuronalen Gewebetypen einen nachweisbaren Marker, bevorzugt einen Fluoreszenzmarker, exprimiert, der von einem anderen Gewebetyp des bi- oder multidifferenzierten neuronalen Gewebes nicht exprimiert wird.

14. Das bi- oder multi-differenzierte neuronale Gewebe nach einem der Ansprüche 12 oder 13, in Form eines globulären Körpers oder in Form eines Gewebeschnitts.

15. Ein Verfahren zum Testen oder Screenen einer Kandidatenverbindung auf Beeinflussung der Differenzierung von bi- oder multi-differenziertem neuronalem Gewebe, umfassend das Kontaktieren von Zellen oder einem neuronalen Gewebe in einem Verfahren nach einem der Ansprüche 1 bis 11 mit der Kandidatenverbindung oder das Kontaktieren eines neuronalen Gewebes nach einem der Ansprüche 12 bis 14 mit der Kandidatenverbindung und das Aufrechterhalten des kontaktierten Gewebes in Kultur, und das Beobachten von Differenzierungsveränderungen im Gewebe im Vergleich zu besagtem Gewebe ohne Kontakt mit der Kandidatenverbindung.

16. Verwendung eines Kits in einem Verfahren nach einem der Ansprüche 1 bis 11, wobei das Kit einen ventralen Differenzierungsinduktor und einen dorsalen Differenzierungsinduktor umfasst, bevorzugt einen WNT-Inhibitor und/oder einen SHH-Verstärker als ventralen Differenzierungsinduktor und bevorzugt einen SHH-Inhibitor als dorsalen Differenzierungsinduktor.

## Revendications

1. Un procédé *in vitro* de production d'un tissu neuronal bi- ou multi-différencié comportant au moins deux types de tissus neuronaux comprenant les étapes de fourniture d'un premier tissu neuronal qui est développé à un stade de différenciation d'intérêt; fourniture d'un second tissu neuronal qui est développé à un stade de différenciation d'intérêt différent du stade de différenciation d'intérêt du premier tissu neuronal, dans lequel le volume du premier et/ou du second tissu est d'au moins 6x10⁶µm³; placement desdits premier et second tissus neuronaux à une proximité suffisante pour la fusion par croissance; permettre aux premier et second tissus neuronaux de croître et de fusionner l'un avec l'autre; produisant ainsi un tissu neuronal bi- ou multi-différencié comprenant les premier et second tissus neuronaux avec différents stades de différenciation.

2. Un procédé *in vitro* de production d'un tissu neuronal bi- ou multi-différencié comportant au moins deux types de tissus neuronaux comprenant les étapes de fourniture d'un premier tissu neuronal qui est développé à un stade de différenciation d'intérêt; fourniture d'un second tissu neuronal qui est développé à un stade de différenciation d'intérêt différent du stade de différenciation d'intérêt du premier tissu neuronal; placement desdits premier et second tissus neuronaux à une proximité suffisante pour la fusion par croissance; permettre aux premier et second tissus neuronaux de croître et de fusionner l'un avec l'autre; produisant ainsi un tissu neuronal bi- ou multi-différencié comprenant les premier et second tissus neuronaux avec différents stades de différenciation; dans lequel le premier et/ou le second tissu neuronal est cultivé dans un hydrogel, et/ou dans une matrice extracellulaire (MEC) ou tout composant de celle-ci sélectionné parmi le collagène, la laminine, l'en-tactine, et le protéoglycane héparane-sulfate ou toute combinaison de ceux-ci, et/ou dans lequel les tissus pendant la fusion sont cultivés dans un hydrogel, et/ou dans une matrice extracellulaire (MEC) ou tout composant de celle-ci sélectionné parmi le collagène, la laminine, l'entactine, et le protéoglycane héparane-sulfate ou toute combinaison de ceux-ci.

3. Le procédé selon la revendication 1 ou 2, dans lequel le premier et/ou le second tissu neuronal, de préférence les deux, sont dérivés d'une culture _in vitro_ de cellules souches pluripotentes, en particulier de cellules souches pluripotentes induites.

4. Le procédé selon la revendication 1 ou 2 ou 3, comprenant le développement du premier tissu neuronal à un stade de différenciation d'intérêt séparé du développement du second tissu neuronal à un stade de différenciation d'intérêt différent.

5. Le procédé selon l'une quelconque des revendications 1 à 4, dans lequel au moins le premier et/ou le second tissu neuronal comprend des cellules progénitrices neuronales et des neurones.

6. Le procédé selon la revendication 5, dans lequel les stades de différenciation du premier tissu neuronal comprennent un tissu progéniteur du cerveau antérieur ventral ou un tissu du télencéphale rostro-ventral.

7. Le procédé selon la revendication 5 ou 6, dans lequel les stades de différenciation du second tissu neuronal comprennent un tissu du cerveau antérieur dorsal ou un neuroectoderme sans différenciation vers un cerveau antérieur ventral ou dorsal.

8. Le procédé selon l'une quelconque des revendications 1 à 7, dans lequel les étapes de placement desdits premier et second tissus neuronaux à une proximité suffisante pour la fusion par croissance et permettant aux premier et second tissus neuronaux de croître et de fusionner l'un avec l'autre sont réalisées en culture en suspension.

9. Le procédé selon l'une quelconque des revendications 1 à 8, dans lequel le tissu neuronal bi- ou multi-différencié est différencié de manière à développer une plaque neurale.

10. Le procédé selon l'une quelconque des revendications 1 à 9, dans lequel les premier et second tissus neuronaux sont cultivés jusqu'à une taille d'au moins 100 µm, de préférence au moins 150 µm, particulièrement préféré au moins 200 µm.

11. Le procédé selon l'une quelconque des revendications 1 à 10, dans lequel le premier et/ou le second tissu neuronal exprime un marqueur détectable qui n'est pas exprimé par l'autre du groupe des premier et second tissus neuronaux.

12. Un tissu neuronal bi- ou multi-différencié comportant au moins deux types de tissus neuronaux de différents stades de différenciation, dans lequel au moins un des types de tissus neuronaux comprend un tissu neuronal ventral et au moins un autre type de tissu neuronal est substantiellement dépourvu de caractère ventral mais contenant des cellules ayant migré à partir du tissu neuronal ventral constituant pas plus de 5% des cellules du tissu substantiellement non-ventral et le tissu bi- ou multi-différencié ayant une taille de 100 µm à 10 mm dans sa dimension la plus longue.

13. Le tissu neuronal bi- ou multi-différencié selon la revendication 12, dans lequel au moins un des types de tissus neuronaux exprime un marqueur détectable, de préférence un marqueur de fluorescence, non exprimé par un autre type de tissu du tissu neuronal bi- ou multi-différencié.

14. Le tissu neuronal bi- ou multi-différencié selon l'une quelconque des revendications 12 ou 13, sous forme globulaire ou sous forme d'une tranche de tissu.

15. Un procédé d'essai ou de criblage d'un composé candidat pour influencer la différenciation d'un tissu neuronal bi- ou multi-différencié, comprenant la mise en contact de cellules ou d'un tissu neuronal dans un procédé selon l'une quelconque des revendications 1 à 11 avec le composé candidat ou la mise en contact d'un tissu neuronal selon l'une quelconque des revendications 12 à 14 avec le composé candidat et le maintien dudit tissu contacté en culture, et l'observation de tout changement de différenciation dans le tissu par rapport audit tissu sans contact par ledit composé candidat.

16. Utilisation d'un kit dans un procédé selon l'une quelconque des revendications 1 à 11, dans lequel le kit comprend un inducteur de différenciation ventrale et un inducteur de différenciation dorsale, de préférence un inhibiteur de WNT et/ou un activateur de SHH comme inducteur de différenciation ventrale et de préférence un inhibiteur de SHH comme inducteur de différenciation dorsale.
